(19) Europäisches Patentamt
European Patent Office
Office européen des brevets

(11) Publication number: **0 253 501 A2**

(12) **EUROPEAN PATENT APPLICATION**

(21) Application number: **87305244.3**

(22) Date of filing: **12.06.87**

(51) Int. Cl.4: **C07D 265/30**, C07D 295/02, C07F 7/08, C07D 295/06, C07C 61/39, C07C 69/753, C07D 295/18, C07C 49/657, C07C 35/21, A01N 33/02, A01N 55/00

The title of the invention has been amended (Guidelines for Examination in the EPO, A-III, 7.3).

(30) Priority: **16.07.86 GB 8617373**
**24.09.86 GB 8622965**

(43) Date of publication of application: **20.01.88 Bulletin 88/03**

(84) Designated Contracting States: **AT BE CH DE ES FR GB GR IT LI LU NL SE**

(71) Applicant: **IMPERIAL CHEMICAL INDUSTRIES PLC**
**Imperial Chemical House Millbank**
**London SW1P 3JF(GB)**

(72) Inventor: **Anthony, Vivienne Margaret**
**4 The Croft**
**Maidenhead Berkshire(GB)**
Inventor: **Urch, Christopher John**
**40 Dalcross Crown Wood**
**Bracknell Berkshire(GB)**
Inventor: **Worthington, Paul Anthony**
**4 Oakhurst Road Maidenhead**
**Court Park Maidenhead Berkshire(GB)**

(74) Representative: **Alner, Henry Giveen Hamilton et al**
**Imperial Chemical Industries PLC Legal Department: Patents P.O. Box 6 Bessemer Road**
**Welwyn Garden City Herts, AL7 1HD(GB)**

(54) **N-disubstituted cycloalkylmethyl amines useful as fungicides.**

(57) Compounds having plant fungicidal properties are of formula:

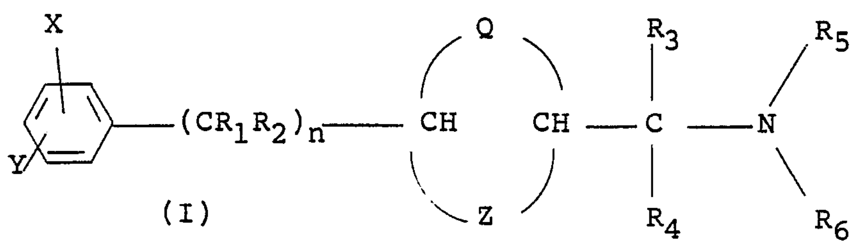

(I)

and stereoisomers thereof, wherein $R_1$ and $R_2$, each represent a hydrogen atom, a halogen atom, or an alkyl group containing from 1 to 4 carbon atoms, $R_3$ and $R_4$ each represent a hydrogen or an alkyl group containing from 1 to 4 carbon atoms, $R_5$ and $R_6$ each represent an alkyl group containing from 1 to 4 carbon atoms or $R_5$ and $R_6$ together with the adjacent nitrogen atom form a heterocyclic ring which may optionally

EP 0 253 501 A2

contain an additional heteroatom, X and Y each represent a hydrogen atom, a halogen atom, or an alkyl, cycloalkyl, cycloalkylalkyl, alkenyl, alkynyl, aryl, aralkyl, alkoxy or aryloxy group, n has the value 0 or 1 and Q and Z are bridging groups as defined.

# TERTIARY AMINE COMPOUNDS

This invention relates to tertiary amine compounds containing a cyclobutane, cyclopentane or cyclohexane ring which are useful as fungicides, to processes for preparing the compounds, to fungicidal compositions containing them, and to methods of using them to combat fungi, especially fungal infections in plants.

According to the invention there are provided compounds having the general formula (I):

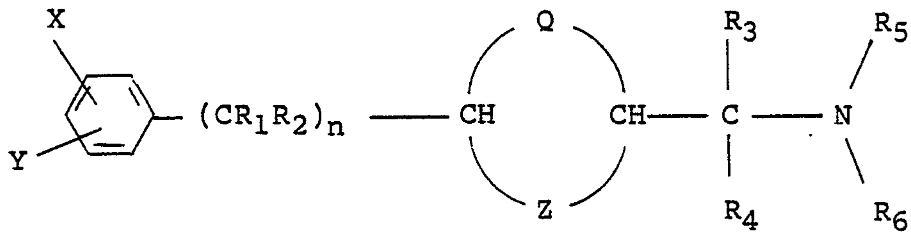

(I)

and stereoisomers thereof, wherein $R_1$ and $R_2$ each represent a hydrogen atom, a halogen atom, or an alkyl group containing from 1 to 4 carbon atoms, $R_3$ and $R_4$ each represent a hydrogen atom or an alkyl group containing from 1 to 4 carbon atoms, $R_5$ and $R_6$ each represent an alkyl group containing from 1 to 4 carbon atoms or $R_5$ and $R_6$ together with the adjacent nitrogen atom form a heterocyclic ring which may optionally contain an additional hetero atom, X and Y each represent a hydrogen atom, a halogen atom, or an alkyl, cycloalkyl, cycloalkylalkyl, alkenyl, alkynyl, aryl, aralkyl, alkoxy or aryloxy group, or a

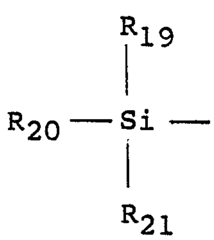

group wherein $R_{19}$, $R_{20}$ and $R_{21}$ can be an alkyl, alkenyl, alkynyl, cycloalkyl, cycloalkylalkyl, aryl or aralkyl group, n has the value 0 or 1 and Q and Z are bridging groups as hereinafter defined; and acid addition salts thereof.

The bridging group Q, subjected to the constraints referred to below, may have one of the following values $Q_1$, $Q_2$, $Q_3$ or $Q_4$:

$Q_1$ =

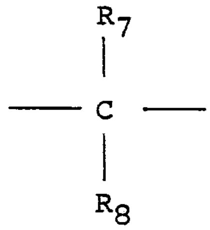

$Q_2$ =

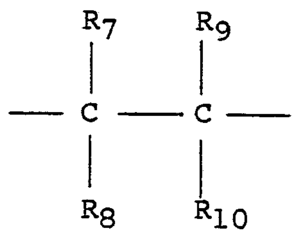

$Q_3$ =

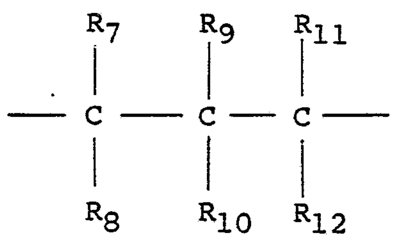

$Q_4$ =

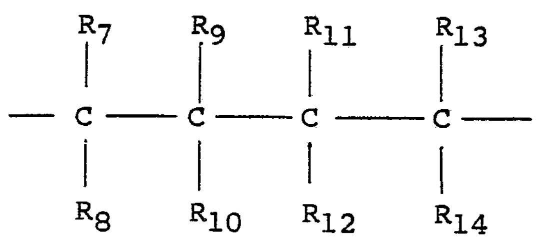

wherein $R_7$ to $R_{14}$ each independently represent a hydrogen atom, a halogen atom, a hydroxyl group or an alkyl group containing from 1 to 4 carbon atoms.

The bridging group Z, subject to the constraints referred to below, may be a covalent linkage, denoted by $Z_o$, or may have one of the following values $Z_1$, or $Z_2$:

$Z_1$ =

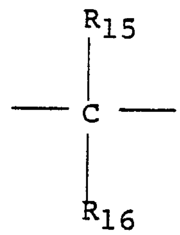

$Z_2$ =

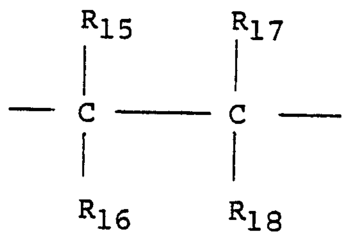

wherein $R_{15}$ to $R_{18}$ each independently have the same significance as $R_7$ to $R_{14}$ above. The identities of the bridging groups Q and Z are subject to the following constraints:

(i) when Z has the value $Z_o$, Q may not have the value $Q_1$;

(ii) the total number of carbon atoms forming part of the ring structure in formula (I) and contributed by the groups Q and Z must not exceed 4.

The compounds of the invention are generally obtained in the form of mixtures of geometric isomers. However, these and other mixtures of optical isomers can be separated into individual isomers by methods in the art and such isomers constitute a part of the present invention.

When $R_5$ and $R_6$, together with the adjacent N-atom, represent a heterocyclic ring this may be, for example, a piperidine, morpholine, thiomorpholine, pyrrolidine or piperazine ring and any of these rings may bear substitutents such as one or more $C_{1-4}$ alkyl groups or a phenyl group, or a hydroxyalkyl group.

Alkyl groups containing from 1 to 4 carbon atoms represented by $R_1$, $R_2$, $R_3$, $R_4$ and $R_7$ to $R_{18}$ may be either straight or branched chain groups, for example methyl, ethyl, n-propyl, iso-propyl, n-butyl, sec-butyl, iso-butyl, tert-butyl. Alkyl and alkoxy groups represented by X and Y and $R_{19}$ to $R_{21}$ when alkyl may be either straight or branched chain groups containing from 1 to 6 carbon atoms; cycloalkyl groups represented by X and Y and $R_{19}$ to $R_{21}$ may be $C_{3-6}$ cycloalkyl groups eg. cyclopropyl, cyclobutyl, cyclopentyl or cyclohexyl groups; or cycloalkylalkyl groups, eg. $C_4$-$C_9$ cycloalkylalkyl groups.

Halogen atoms represented by $R_1$ and $R_2$, $R_7$ to $R_{18}$ and X and Y may be fluorine, chlorine or bromine atoms.

Alkenyl and alkynyl groups represented by X and Y may contain from 2 to 6 carbon atoms and for $R_{19}$ to $R_{21}$ from 2 to 4 carbon atoms.

Examples of X and Y when these are aryl, aralkyl, aryloxy or aralkoxy groups are phenyl, benzyl, phenoxy and benzyloxy. Examples of $R_{19}$ to $R_{21}$ when these are aryl and aralkyl are phenyl and benzyl. These rings may be substituted with halogen (eg, fluorine, chlorine, or bromine), $C_{1-6}$ alkyl [eg, methyl, ethyl, propyl (n-or iso-propyl) and butyl (n-, sec-, iso, or t-butyl)], $C_{1-6}$ alkoxy (eg, methoxy, ethoxy, propoxy and butoxy) halo-$C_{1-6}$ -alkoxy (eg, tri-fluoromethoxy), halo-$C_{1-6}$ -alkyl (eg, trifluoromethyl), nitro, phenyl and phenoxy. Examples of X and Y when these are a silyl group are trimethylsilyl, triethylsilyl, t -butyldimethylsilyl, phenyldimethylsilyl and allyldimethylsilyl. The phenyl ring may thus be unsubstituted or substituted with ring substituents as defined above. The X and Y substituents may be at the 2-, 3-or 4-positions of the phenyl ring and the 4-position is preferred.

According to one particular aspect of the invention, there are provided compounds containing a cyclobutane ring and having the general formula (I):

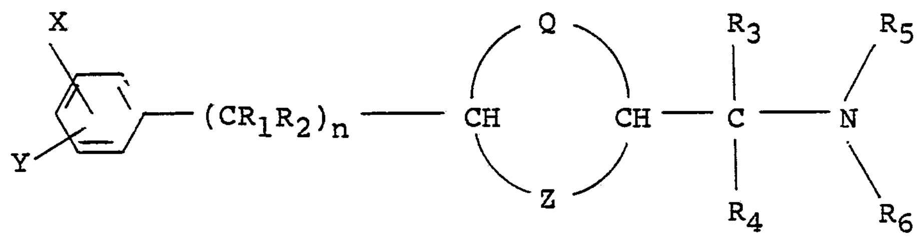

(I)

and stereoisomers and addition salts thereof, wherein either Q has the value $Q_2$ and Z has the value $Z_o$ as previously defined, or Q has the value $Q_1$ and Z has the value $Z_1$ as previously defined, the remaining symbols having the same significance as before. These two alternatives correspond to compounds in which the molecular residues:

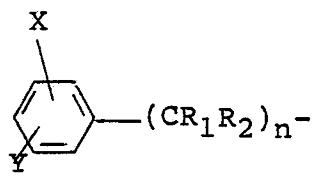

and

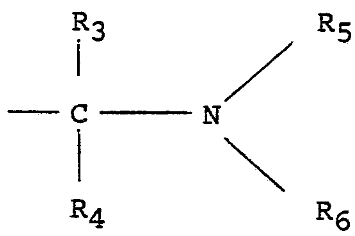

are attached to the cyclobutane ring in the 1,2-and 1,3-positions respectively.

According to another particular aspect of the invention, there are provided compounds containing a cyclopentane ring and having the general formula (I) above, wherein Q has the value $Q_3$ and Z has the value $Z_o$, or Q has the value $Q_2$ and Z has the value $Z_1$, as previously defined, the remaining symbols having the same significance as before. These two possibilities correspond to substitution of the cyclopentane ring by the molecular residues referred to above in the 1,2-and 1,3-positions respectively.

According to a further particular aspect of the invention, there are provided compounds containing a cyclohexane ring and having the general formula (I) above, wherein Q has the value $Q_4$ and Z has the value $Z_o$, or Q has the value $Q_3$ and Z has the value $Z_1$, or Q has the value $Q_2$ and Z has the value $Z_2$ as previously defined, the remaining symbols having the same significance as before. These three possibilities correspond to 1,2-, 1,3-or 1,4-substitution respectively of the cyclohexane ring by the two molecular residues referred to previously.

Examples of the compounds of the invention are shown in Tables I to VII. These are 1,3-disubstituted cyclobutanes, 1,3-disubstituted cyclopentanes, 1,2-disubstituted cyclobutanes, 1,2-disubstituted cyclopentanes, 1,3-disubstituted cyclohexanes, 1,2-disubstituted cyclohexanes and 1,4-disubstituted cyclohexanes respectively which conform to formula I.

## TABLE I

1,3-DISUBSTITUTED CYCLOBUTANES

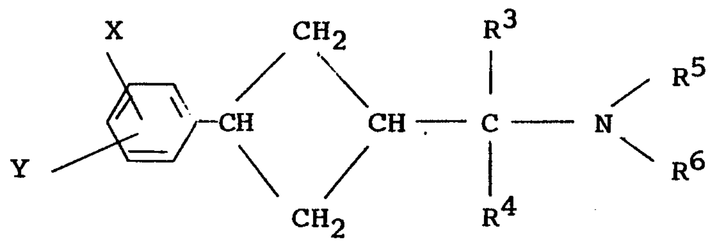

| COMPOUND NO | X | Y | $R^3$ | $R^4$ | 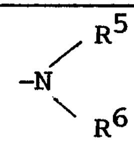 | mp/bp (°C) | COMMENTS |
|---|---|---|---|---|---|---|---|
| 1.1 | 4-t-$C_4H_9$ | H | H | H | 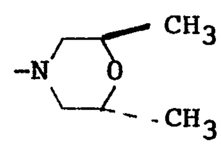 | oil | trans Ar/$CH_2$ |
| 2.1 | 4-t-$C_4H_9$ | H | H | H | 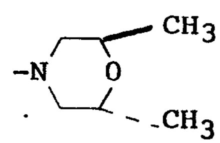 | oil | cis Ar/$CH_2$ |

TABLE I (cont)

1,3-DISUBSTITUTED CYCLOBUTANES

| COMPOUND NO | X | Y | $R^3$ | $R^4$ | 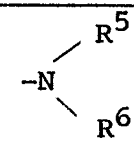 | mp/bp (°C) | COMMENTS |
|---|---|---|---|---|---|---|---|
| 3.1 | 4-t-$C_4H_9$ | H | H | H | 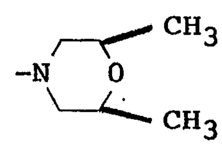 | oil | cis Ar/$CH_2$ |
| 4.1 | 4-t-$C_4H_9$ | H | H | H | 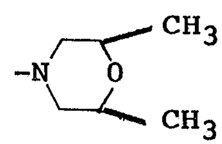 | oil | trans Ar/$CH_2$ |
| 5.1 | 4-t-$C_4H_9$ | H | H | H | 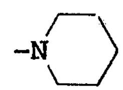 | oil | cis: trans Ar/$CH_2$ = 2:3 |
| 6.1 | H | H | H | H | 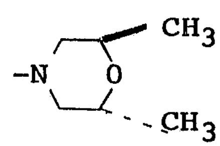 | oil | trans Ar/$CH_2$ |
| 7.1 | H | H | H | H | 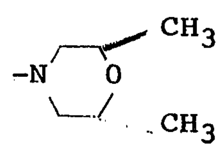 | oil | cis Ar/$CH_2$ |

TABLE I (cont)

1,3-DISUBSTITUTED CYCLOBUTANES

| COMPOUND NO | X | Y | $R^3$ | $R^4$ | 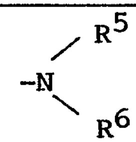 | mp/bp (°C) | COMMENTS |
|---|---|---|---|---|---|---|---|
| 8.1 | H | H | H | H | 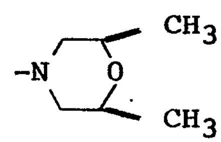 | oil | trans $Ar/CH_2$ |
| 9.1 | H | H | H | H | 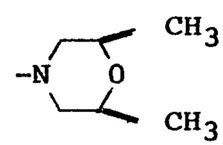 | oil | cis $Ar/CH_2$ |
| 10.1 | 4-i-$C_3H_7$ | H | H | H | 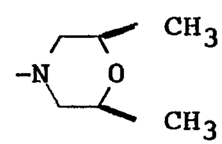 | oil | cis:trans $Ar/CH_2$ = 1:1 |
| 11.1 | 4-i-$C_3H_7$ | H | H | H | 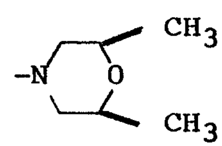 |  | cis $Ar/CH_2$ |
| 12.1 | 4-i-$C_3H_7$ | H | H | H | 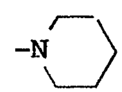 |  | cis $Ar/CH_2$ |

TABLE I (cont)

1,3-DISUBSTITUTED CYCLOBUTANES

| COMPOUND NO | X | Y | $R^3$ | $R^4$ | 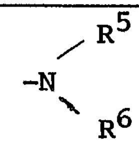 | mp/bp (°C) | COMMENTS |
|---|---|---|---|---|---|---|---|
| 13.1 | 4-i-$C_3H_7$ | H | H | H | 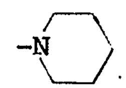 | | trans Ar/$CH_2$ |
| 14.1 | 4-$(CH_3)_3Si$ | H | H | H | 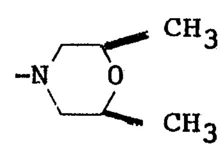 | oil | cis Ar/$CH_2$ |
| 15.1 | 4-$(CH_3)_3Si$ | H | H | H | 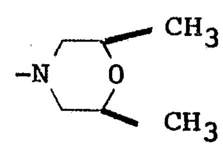 | oil | trans Ar/$CH_2$ |
| 16.1 | 4-$(CH_3)_3Si$ | H | H | H | 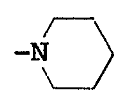 | | cis Ar/$CH_2$ |
| 17.1 | 4-$(CH_3)_3Si$ | H | H | H | 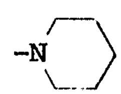 | | trans Ar/$CH_2$ |

TABLE I (cont)

1,3-DISUBSTITUTED CYCLOBUTANES

| COMPOUND NO | X | Y | $R^3$ | $R^4$ | 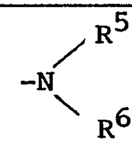 | mp/bp (°C) | COMMENTS |
|---|---|---|---|---|---|---|---|
| 18.1 | $3-(CH_3)_3Si$ | H | H | H | 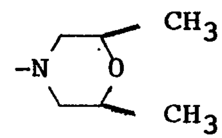 | | cis $Ar/CH_2$ |
| 19.1 | $3-(CH_3)_3Si$ | H | H | H | 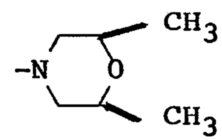 | | trans $Ar/CH_2$ |
| 20.1 | $3-(CH_3)_3Si$ | H | H | H | 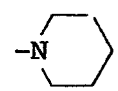 | | cis $Ar/CH_2$ |
| 21.1 | $3-(CH_3)_3Si$ | H | H | H | 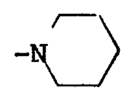 | | trans $Ar/CH_2$ |

TABLE II

1,3-DISUBSTITUTED CYCLOPENTANES

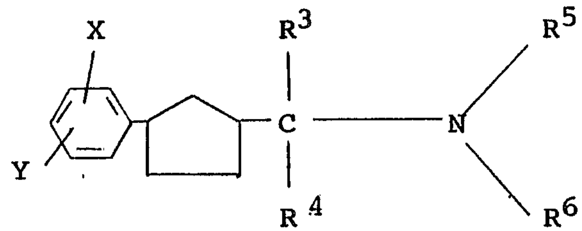

| COMPOUND NO | X | Y | $R^3$ | $R^4$ | 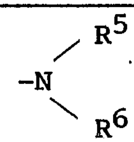 | mp/bp (°C) | COMMENTS |
|---|---|---|---|---|---|---|---|
| 1.2 | 4-i-$C_3H_7$ | H | H | H | 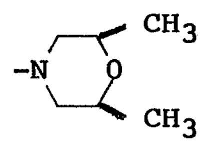 | oil | cis:trans $Ar/CH_2$ = 1:1 |
| 2.2 | 4-i-$C_3H_7$ | H | H | H | 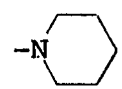 | | |
| 3.2 | 4-t-$C_4H_9$ | H | H | H | 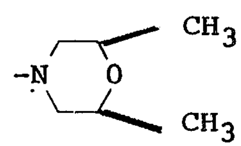 | oil | cis:trans $Ar/CH_2$ = 1:1 |

TABLE II (cont)

1,3-DISUBSTITUTED CYCLOPENTANES

| COMPOUND NO | X | Y | $R^3$ | $R^4$ | 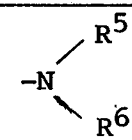 | mp/bp (°C) | COMMENTS |
|---|---|---|---|---|---|---|---|
| 4.2 | 4-t-$C_4H_9$ | H | H | H | 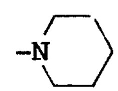 | | |
| 5.2 | 4-i-$C_4H_9$ | H | H | H | 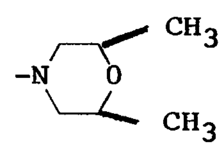 | oil | cis:trans Ar/$CH_2$ = 1:1 |
| 6.2 | 4-n-$C_4H_9$ | H | H | H | 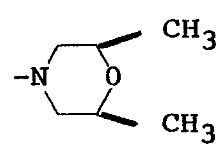 | oil | cis:trans Ar/$CH_2$ = 1:1 |

TABLE II (cont)

1,3-DISUBSTITUTED CYCLOPENTANES

| COMPOUND NO | X | Y | $R^3$ | $R^4$ | 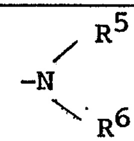 | mp/bp (°C) | COMMENTS |
|---|---|---|---|---|---|---|---|
| 7.2 | 4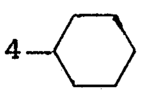 | H | H | H | 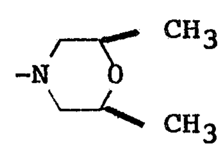 | oil | cis:trans $Ar/CH_2$ = 1.1 |
| 8.2 | 4-s-$C_4H_9$ | H | H | H | 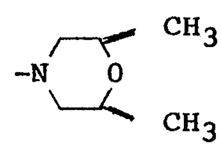 | oil | cis:trans $Ar/CH_2$ = 1:1 |
| 9.2 | 4-s-$C_4H_9$ | H | H | H | 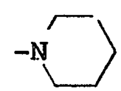 | | |

TABLE II (cont)

1,3-DISUBSTITUTED CYCLOPENTANES

| COMPOUND NO | X | Y | $R^3$ | $R^4$ | 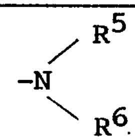 | mp/bp (°C) | COMMENTS |
|---|---|---|---|---|---|---|---|
| 10.2 | $4-(CH_3)_3Si$ | H | H | H | 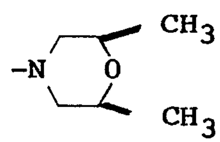 | | |
| 11.2 | $4-(CH_3)_3Si$ | H | H | H | 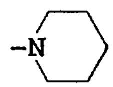 | | |
| 12.2 | $3-(CH_3)_3Si$ | H | H | H | 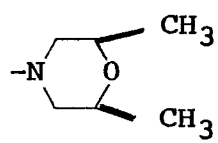 | | |
| 13.2 | $3-(CH_3)_3Si$ | H | H | H | 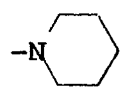 | | |

TABLE II (cont)

1,3-DISUBSTITUTED CYCLOPENTANES

| COMPOUND NO | X | Y | $R^3$ | $R^4$ | 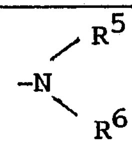 | mp/bp (°C) | COMMENTS |
|---|---|---|---|---|---|---|---|
| 14.2 | 4-Cl | H | H | H | 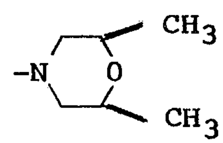 | oil | cis:trans Ar/$CH_2$ = 1:1 |
| 15.2 | 4-Cl | H | H | H | 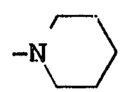 | | |

TABLE III

1,2-DISUBSTITUTED CYCLOBUTANES

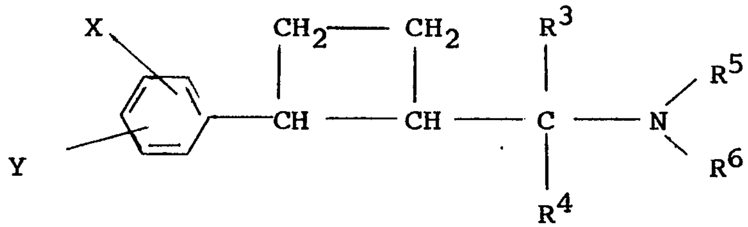

| COMPOUND NO | X | Y | $R^3$ | $R^4$ | 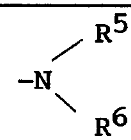 | mp/bp (°C) | COMMENTS |
|---|---|---|---|---|---|---|---|
| 1.3 | 4-$\underline{t}$-$C_4H_9$ | H | H | H | 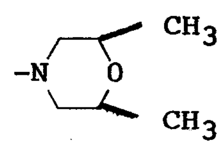 | oil | $\underline{trans}$ Ar/$CH_2$ |
| 2.3 | 4-$\underline{t}$-$C_4H_9$ | H | H | H | 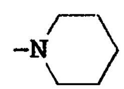 | | |
| 3.3 | 4-$\underline{i}$-$C_3H_7$ | H | H | H | 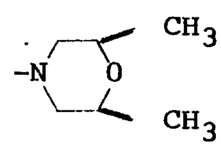 | | |

TABLE III (cont)

1,2-DISUBSTITUTED CYCLOBUTANES

| COMPOUND NO | X | Y | $R^3$ | $R^4$ | 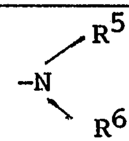 | mp/bp (°C) | COMMENTS |
|---|---|---|---|---|---|---|---|
| 4.3 | 4-i-$C_3H_7$ | H | H | H | 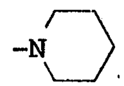 | | |
| 5.3 | 4-$(CH_3)_3Si$ | H | H | H | 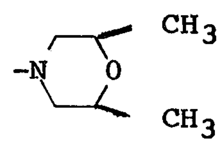 | | |
| 6.3 | 4-$(CH_3)_3Si$ | H | H | H | 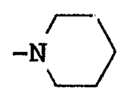 | | |
| 7.3 | 3-$(CH_3)_3Si$ | H | H | H | 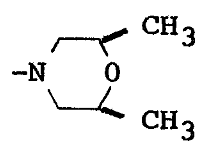 | | |
| 8.3 | 3-$(CH_3)_3Si$ | H | H | H | 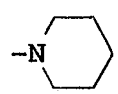 | | |

## TABLE IV

### 1,2-DISUBSTITUTED CYCLOPENTANES

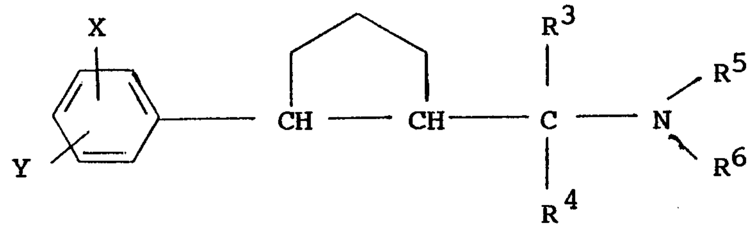

| COMPOUND NO | X | Y | $R^3$ | $R^4$ | 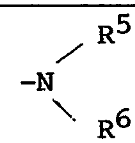 | mp/bp (°C) | COMMENTS |
|---|---|---|---|---|---|---|---|
| 1.4 | 4-t-$C_4H_9$ | H | H | H | 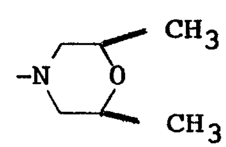 | | |
| 2.4 | 4-t-$C_4H_9$ | H | H | H | 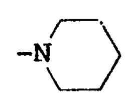 | | |
| 3.4 | 4-i-$C_3H_7$ | H | H | H | 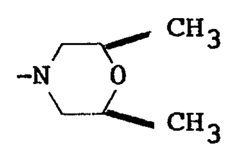 | | |

## TABLE IV (cont)

## 1,2-DISUBSTITUTED CYCLOPENTANES

| COMPOUND NO | X | Y | $R^3$ | $R^4$ | 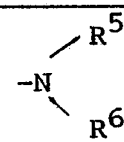 | mp/bp (°C) | COMMENTS |
|---|---|---|---|---|---|---|---|
| 4.4 | 4-$\underline{i}$-$C_3H_7$ | H | H | H | 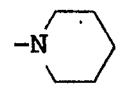 | | |
| 5.4 | 4-$(CH_3)_3Si$ | H | H | H | 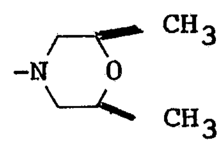 | | |
| 6.4 | 4-$(CH_3)_3Si$ | H | H | H | 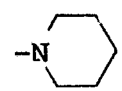 | | |
| 7.4 | 3-$(CH_3)_3Si$ | H | H | H | 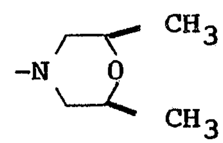 | | |
| 8.4 | 3-$(CH_3)_3Si$ | H | H | H | 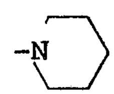 | | |

TABLE IV (cont)

1,2-DISUBSTITUTED CYCLOPENTANES

| COMPOUND NO | X | Y | $R^3$ | $R^4$ | 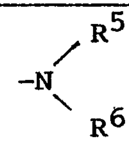 | mp/bp (°C) | COMMENTS |
|---|---|---|---|---|---|---|---|
| 9.4 | 4-Cl | H | H | H | 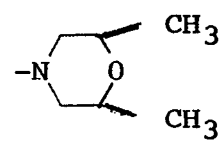 | | |
| 10.4 | 4-Cl | H | H | H | 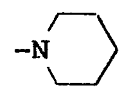 | | |

# TABLE V

## 1,3-DISUBSTITUTED CYCLOHEXANES

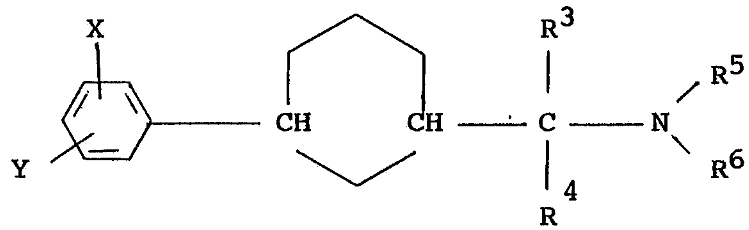

| COMPOUND NO | X | Y | $R^3$ | $R^4$ | 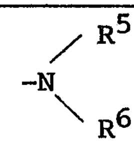 | mp/bp (°C) | COMMENTS |
|---|---|---|---|---|---|---|---|
| 1.5 | 4-t-$C_4H_9$ | H | H | H | 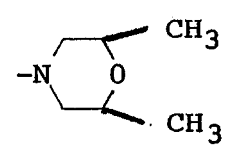 | | |
| 2.5 | 4-t-$C_4H_9$ | H | H | H | 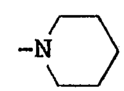 | | |
| 3.5 | 4-i-$C_3H_7$ | H | H | H | 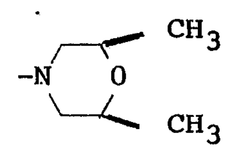 | | |

TABLE V (cont)

1,3-DISUBSTITUTED CYCLOHEXANES

| COMPOUND NO | X | Y | $R^3$ | $R^4$ | 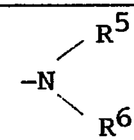 | mp/bp (°C) | COMMENTS |
|---|---|---|---|---|---|---|---|
| 4.5 | 4-i-$C_3H_7$ | H | H | H | 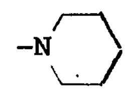 | | |
| 5.5 | 4-Cl | H | H | H | 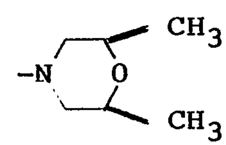 | | |
| 6.5 | 4-Cl | H | H | H | 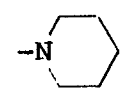 | | |
| 7:5 | 4-$(CH_3)_3Si$ | H | H | H | 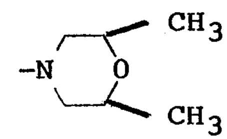 | | |
| 8.5 | 4-$(CH_3)_3Si$ | H | H | H | 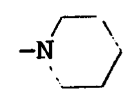 | | |

TABLE V (cont)

1,3-DISUBSTITUTED CYCLOHEXANES

| COMPOUND NO | X | Y | $R^3$ | $R^4$ | 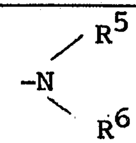 | mp/bp (°C) | COMMENTS |
|---|---|---|---|---|---|---|---|
| 9.5 | 3-$(CH_3)_3Si$ | H | H | H | 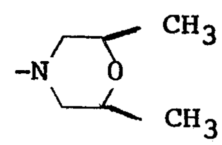 | | |
| 10.5 | 3-$(CH_3)_3Si$ | H | H | H | 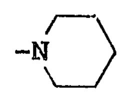 | | |

## TABLE VI

1,2-DISUBSTITUTED CYCLOHEXANES

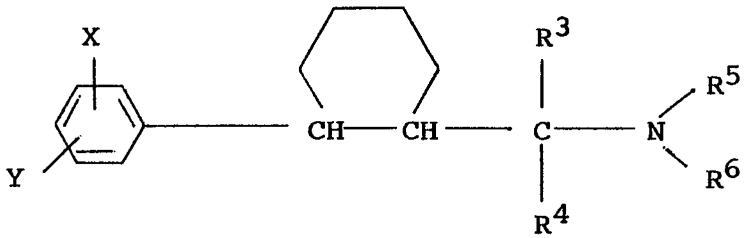

| COMPOUND NO | X | Y | $R^3$ | $R^4$ | 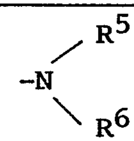 | mp/bp (°C) | COMMENTS |
|---|---|---|---|---|---|---|---|
| 1.6 | 4-t-$C_4H_9$ | H | H | H | 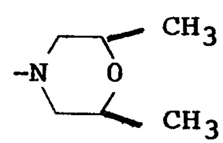 | | |
| 1.7 | 4-t-$C_4H_9$ | H | H | H | 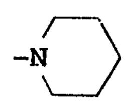 | | |
| 3.6 | 4-i-$C_3H_7$ | H | H | H | 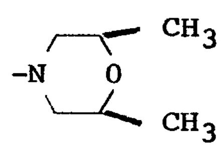 | | |

TABLE VI (cont)

1,2-DISUBSTITUTED CYCLOHEXANES

| COMPOUND NO | X | Y | $R^3$ | $R^4$ | 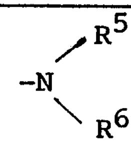 | mp/bp (°C) | COMMENTS |
|---|---|---|---|---|---|---|---|
| 4.6 | 4-$\underline{i}$-$C_3H_7$ | H | H | H | 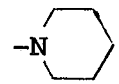 | | |
| 5.6 | 4-Cl | H | H | H | 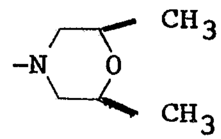 | | |
| 6.6 | 4-Cl | H | H | H | 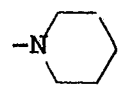 | | |
| 7.6 | 4-$(CH_3)_3Si$ | H | H | H | 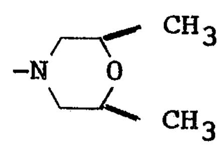 | | |
| 8.6 | 4-$(CH_3)_3Si$ | H | H | H | 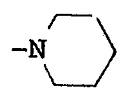 | | |

TABLE VII

1,4-DISUBSTITUTED CYCLOHEXANES

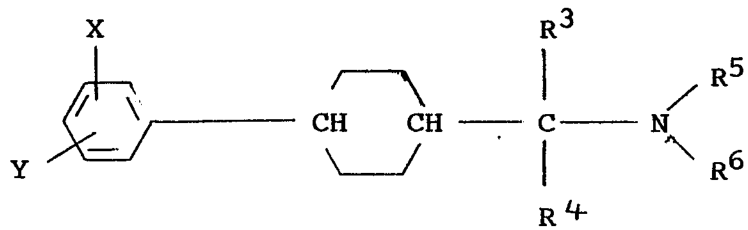

| COMPOUND NO | X | Y | $R^3$ | $R^4$ | 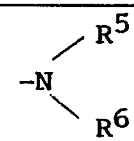 | mp/bp (°C) | COMMENTS |
|---|---|---|---|---|---|---|---|
| 1.7 | 4-t-$C_4H_9$ | H | H | H | 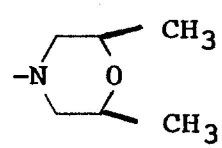 | | |
| 2.7 | 4-t-$C_4H_9$ | H | H | H | 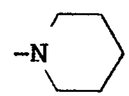 | | |
| 3.7 | 4-i-$C_3H_7$ | H | H | H | 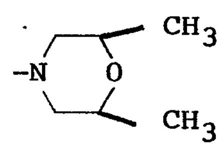 | | |

TABLE VII (cont)

1,4-DISUBSTITUTED CYCLOHEXANES

| COMPOUND NO | X | Y | $R^3$ | $R^4$ | 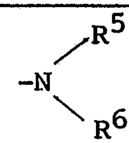 | mp/bp (°C) | COMMENTS |
|---|---|---|---|---|---|---|---|
| 4.7 | 4-i-$C_3H_7$ | H | H | H | 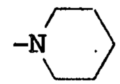 | | |
| 5.7 | 4-Cl | H | H | H | 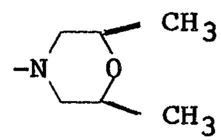 | | |
| 6.7 | 4-Cl | H | H | H | 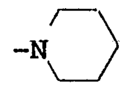 | | |
| 7.7 | 4-$(CH_3)_3Si$ | H | H | H | 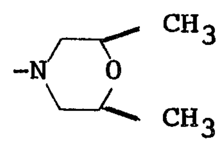 | | |
| 8.7 | 4-$(CH_3)_3Si$ | H | H | H | 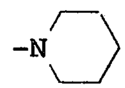 | | |

Compounds of general formula (I) :

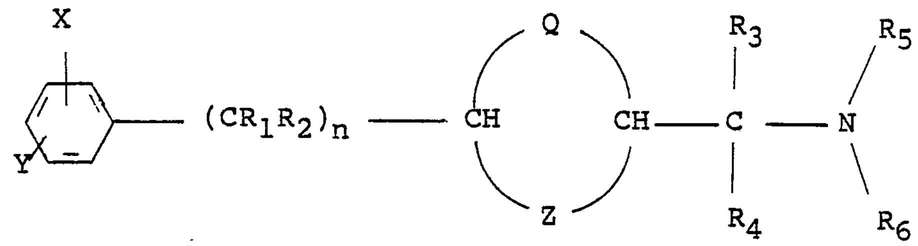

(I)

wherein $R_1$ to $R_6$, Q, X, Y, Z and n are as defined above and where $R_3 = R_4 =$ hydrogen can be prepared by treatment of compounds of general formula (II) :

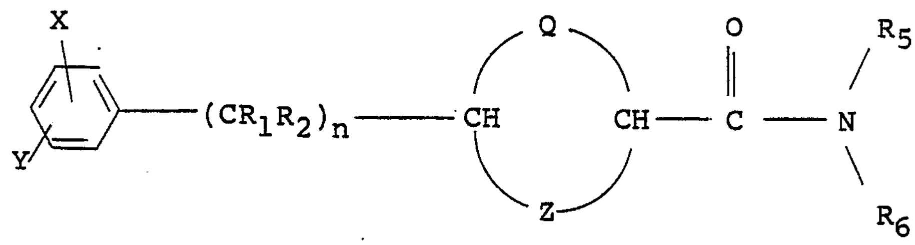

(II)

wherein $R_1$, $R_2$, $R_5$, $R_6$, Q, X, Y, Z and n are as defined above with a reducing agent (usually lithium aluminium hydride) in a convenient solvent such as diethyl ether, tetrahydrofuran or dioxane.

The amides of general formula (II) can be prepared by reacting an acid chloride of general formula (III) :

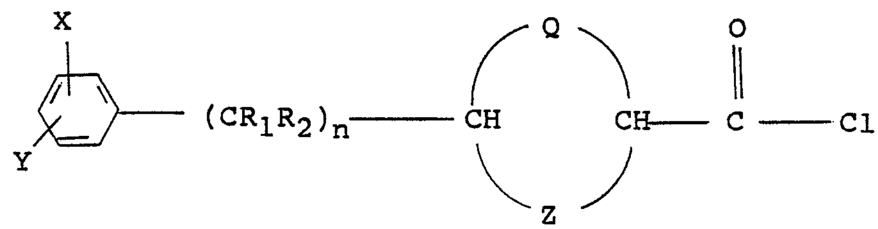

(III)

wherein $R_1$, $R_2$, Q, X, Y, Z and n are as defined above with an amine of general formula (IV) :

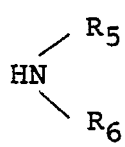

(IV)

wherein $R_5$ and $R_6$ are as defined above, in a convenient solvent such as diethyl ether or tetrahydrofuran.

Acid chlorides of general formula (III) can be prepared by reacting an acid of general formula (V)

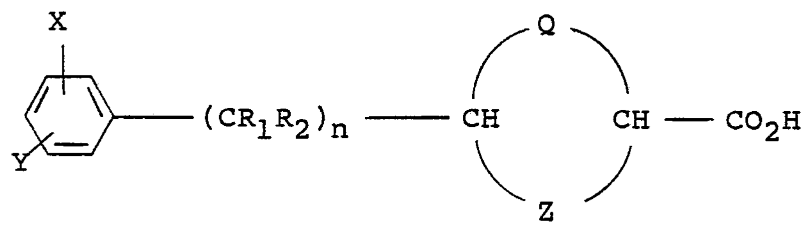

(V)

wherein $R_1$, $R_2$, Q, X, Y, Z and n are as defined above, with the usual chlorinating agents such as thionyl chloride or oxalyl chloride in a convenient solvent such as hexane or dichloromethane.

Acids of general formula (V) can be prepared by hydrolysing esters of general formula (VI) :

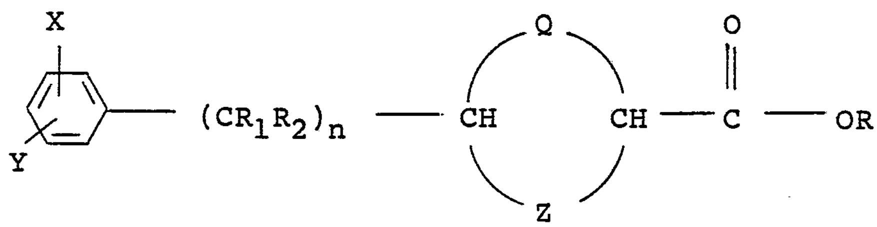

(VI)

wherein $R_1$, $R_2$,. Q, X, Y, Z and n are as defined above and R is alkyl, in the normal manner using either sodium or potassium hydroxide in methanol/water solution at a temperature of 20-60°C.

As an alternative, compounds of general formula (I) where $R_4$ = hydrogen can be prepared by treatment of compounds of general formula (VII) :

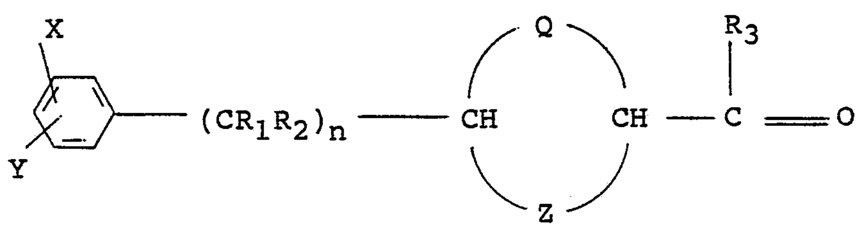

(VII)

wherein $R_1$ to $R_3$, Q, X, Y, Z and n are as defined above, with an amine of general formula (IV) in the presence of a reducing agent such as formic acid, sodium cyanoborohydride or tetrabutylammonium cyanoborohydride.

As a further alternative compounds of general formula (I) can be prepared by treatment of a compound of general formula (VIII) :

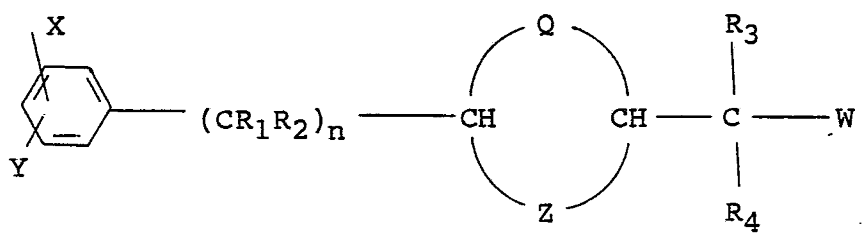

(VIII)

wherein $R_1$ to $R_4$, Q, X, Y, Z and n are as defined above and W is a leaving group such as chlorine, bromine, mesylate or tosylate, with an amine of general formula (IV), in the presence of a convenient solvent such as ethanol or tetrahydrofuran or, preferably, in the absence of a solvent at a temperature of 20-100°C.

Compounds of general formula (VIII) can be prepared by treating an alcohol of general formula (IX) :

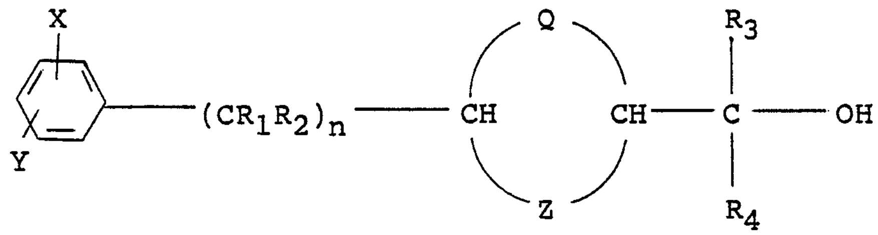

(IX)

wherein $R_1$ to $R_4$, Q, X, Y, Z and n are as defined above, with the usual halogenating agents (eg. phosphorous trichloride, phosphorous pentachloride or phosphorous oxychloride when W = chlorine and phosphorous tribromide, phosphorous pentabromide or phosphorous oxybromide when W = bromine), or mesyl chloride in pyridine (or triethylamine) when W = mesylate, or tosyl chloride in pyridine (or triethylamine) when W = tosylate.

Ketones of general formula (VII) wherein $R_3$ = hydrogen can be prepared from esters of general formula (VI) by treatment with one equivalent of diisobutylaluminium hydride at -78°C in a convenient solvent such as toluene.

As an alternative ketones of general formula (VII) can be prepared from acid chlorides of general formula (III) with a Grignard reagent of general formula (X) :

$R_3MgBr$

(X)

wherein $R_3$ is as defined above at a low temperature (for instance -30°C) in a convenient solvent such as tetrahydrofuran.

As a further alternative ketones of general formula (VII) can be prepared from alcohols of general formula (IX) wherein $R_4$ = hydrogen ~~by~~ oxidation with one of the normal oxidising reagents, for instance chromium trioxide or the Swern modification of the Moffat oxidation (dimethyl sulphoxide, oxalyl chloride followed by triethylamine).

As a further alternative ketones at general formula (VII) can be prepared by hydrolysis of enol ethers of general formula (XI) :

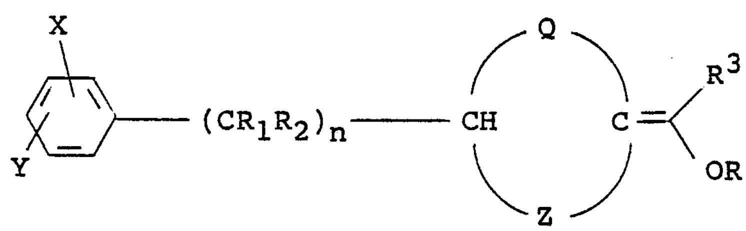

(XI)

wherein R, $R_1$ to $R_3$, Q, X, Y, Z and n are as defined above with aqueous acid, such as dilute hydrochloric acid.

Enol ethers of general formula (XI) can be prepared by treatment of a ketone of general formula (XII) :

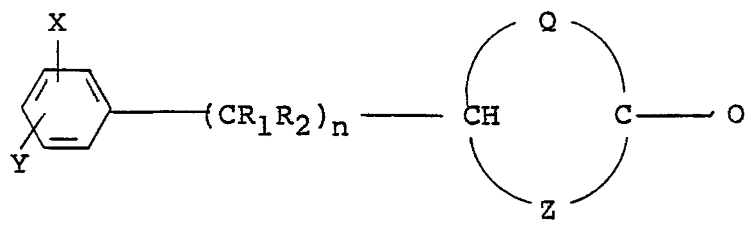

(XII)

wherein $R_1$, $R_2$, Q, X, Y, Z and n are as defined above with a phosphorane of general formula (XIII) :

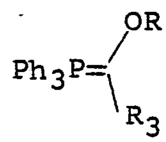

(XIII)

wherein R and $R_3$ are as defined above, or a phosphine oxide anion of general formula (XIV) :

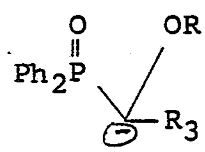

(XIV)

wherein R and $R_3$ are as defined above, in a convenient solvent as described in the literature.

Alcohols of general formual (IX) wherein $R_3 = R_4 =$ hydrogen can be prepared by treatment of an ester of general formula (VI) with a reducing agent (usually lithium aluminium hydride) in a convenient solvent such as diethyl ether or tetrahydrofuran.

As an alternative alcohols of general formula (IX) wherein $R_3 = R_4 =$ alkyl can be prepared by treatment of an ester of general formula (VI) with an excess of a Grignard reagent of general formula (X) or an excess of an alkyl lithium reagent in a convenient solvent such as diethyl ether or tetrahydrofuran.

As a further alternative alcohols of general formula (IX) can be prepared by treatment of ketones of general formula (VII) with a Grignard reagent of general formula (XV) :

$R_4MgBr$

(XV)

wherein $R_4$ is as defined above, or an alkyl lithium reagent in a convenient solvent such as diethyl ether or tetrahydrofuran.

As a further alternative alcohols of general formula (IX) wherein $R_4 =$ hydrogen can be prepared by hydroboration of alkenes of general formula (XVI) :

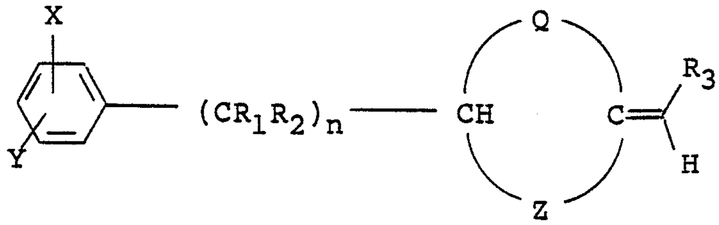

(XVI)

wherein $R_1$ to $R_3$, Q, X, Y, Z and n are as defined above, followed by oxidative workup.

Alkenes of general formula (XVI) can be prepared from the corresponding ketone of general formula (XII) by reaction with a phosphorane of general formula (XVII) :

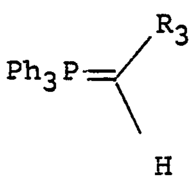

(XVII)

wherein $R_3$ is as defined above, under the conditions of the Wittig reaction or a modification thereof, as described in the literature.

Ketones of general formula (XII) wherein Q = $Q_1$, ($R_7$ = $R_8$ = hydrogen) and Z = $Z_1$, can be prepared by dechlorination of ketones of general formula (XVIII) :

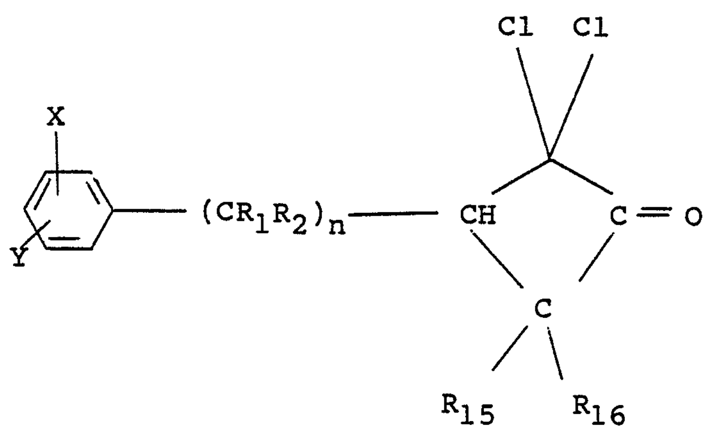

(XVIII)

wherein $R_1$, $R_2$, $R_{15}$, $R_{16}$, X, Y and n are as defined above (but $R_{15}$ and $R_{16}$ represent a hydrogen atom or an alkyl group only), with a reducing reagent such as tributyltin hydride or zinc in acetic acid.

Ketones of general formula (XVIII) can be prepared by treating an alkene of general formula (XIX) :

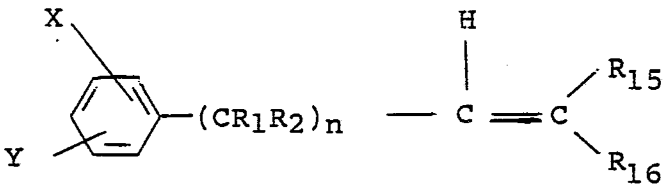

(XIX)

wherein $R_1$, $R_2$, $R_{15}$, $R_{16}$, X, Y and n are as defined immediately above with dichloroketene generated for instance by the action of zinc on trichloroacetyl chloride or the action of triethylamine on dichloroacetyl chloride.

Alkenes of general formula (XIX) can be prepared by methods set out in the literature.

As an alternative ketones of general formula (XII) wherein $Z = Z_1$, ($R_{15}$ = hydrogen and $R_{16}$ = hydrogen or alkyl) and Q = $Q_2$ or $Q_3$ can be prepared by treating enones of general formula (XX) :

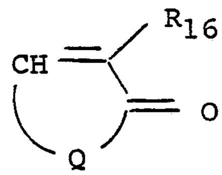

(XX)

wherein $R_{16}$ and Q are as defined immediately above with either an organocuprate reagent of general formula (XXI) :

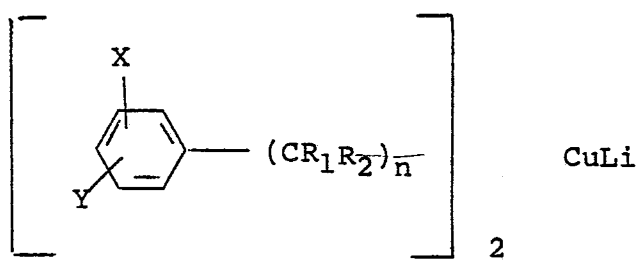

(XXI)

wherein $R_1$, $R_2$, X, Y and n are as defined above or an organozinc reagent of general formula (XXII) :

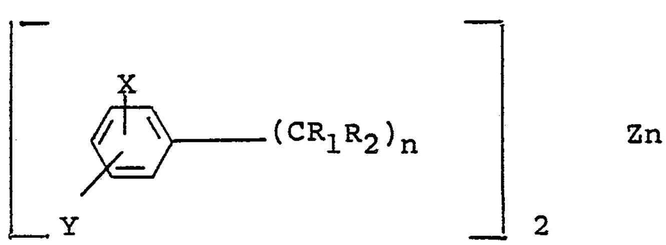

(XXII)

wherein $R_1$, $R_2$, X, Y and n are as defined above, optionally in the presence of a catalytic amount of nickel acetylacetonate.

Enones of general formula (XX) can be prepared as described in the literature.

Organocuprate reagents of general formula (XXI) and organozinc reagents of general formula (XXII) can be prepared by methods set out in the literature.

As a further alternative ketones of general formula (XII) $Z = Z_o$ can be prepared by treatment a of 2-chloroketone of general formula (XXIII) :

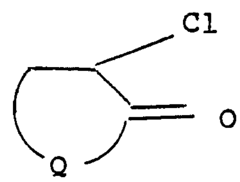

(XXIII)

wherein Q is as defined above with a Grignard reagent of general formula (XXIV) :

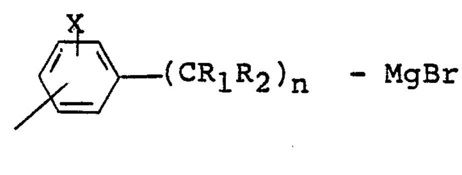

(XXIV)

wherein $R_1$, $R_2$, X, Y and n are as defined above as described in the literature.

Esters of general formula (VI) can be prepared by decarboxylating diesters of general formula (XXV) :

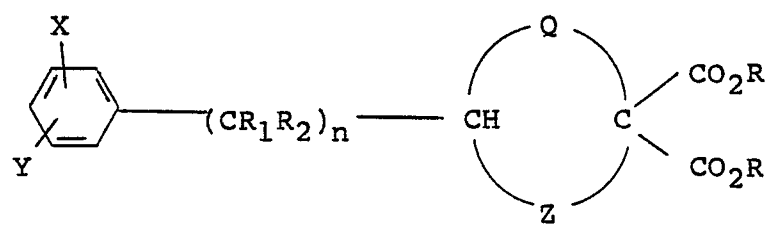

(XXV)

wherein R, $R_1$, $R_2$, Q, X, Y, Z and n are as defined above with standard reagents such as lithium chloride in wet dimethyl-sulphoxide using the conditions of Krapcho (J. Org. Chem. 1978, 43, 138-147).

Diesters of general formula (XXV) can be prepared by reacting a compound of general formula (XXVI) :

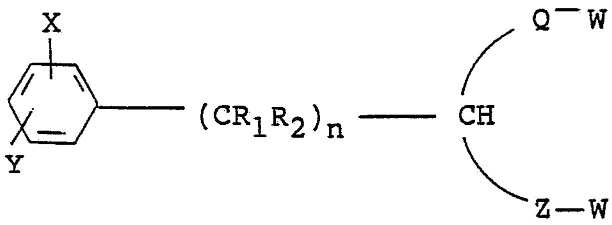

(XXVI)

wherein $R_1$, $R_2$, Q, W, X, Y, Z and n are as defined above, with a convenient base such as sodium hydride or potassium t-butoxide in a convenient solvent such as diethyl ether, tetrahydrofuran or dioxane.

Compounds of general formula (XXVI) can be prepared by standard methods set out in the literature.

As an alternative esters of general formula (VI) wherein Z=Z can be prepared by treating unsaturated esters of general formula (XXVII) :

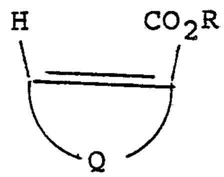

(XXVII)

wherein R and Q are as defined above with an organocuprate reagent of general formula (XXI).

Unsaturated esters of general formula (XXVII) can be prepared by standard methods set out in the literature.

The salts of compounds of the general formula (I) can be prepared from the latter by known methods.

The compounds and their salts are active fungicides, and may be used to control one or more of the following pathogens:

Puccinia recondita, Puccinia striiformis and other rusts on wheat, Puccinia hordei, Puccinia striiformis and other rusts on barley, and rusts on other hosts e.g. coffee, apples, pears, peanuts, vegetables and ornamental plants.

Erysiphe graminis (powdery mildew) on barley and wheat and other powdery mildews on various hosts such as Sphaerotheca fuliginea on cucurbits (e.g. cucumber), Podosphaera leucotricha on apples and Uncinula necator on vines.

Helminthosporium spp., eg. Pyrenophora teres Rhynchosporium spp. and Septoria spp. on cereals eg. Septoria nodorum on wheat.

Cercospora arachidicola on peanuts and other Cercospora species on for example sugar beet, bananas and soya beans Venturia inaequalis (scab) on apples.

Some of the compounds have also shown a broad range of activities against fungi in vitro. They have activity against various post-harvest pathogens on fruit (e.g. Penicillium digatatum and italicum on oranges, Gloeosporium musarum on bananas and Botryris cinerea on grape). Further some of the compounds are active as seed dressings against: Fusarium spp., Septoria spp., Tilletia spp. (i.e. bunt, a seed borne disease of wheat), Ustilago spp., and Helminthosporium spp. on cereals, and Rhizoctonia solani on cotton.

The compounds can move acropetally in the plant tissue. Moreover, the compounds can be volatile enough to be active in the vapour phase against fungi on the plant.

They may also be useful as industrial (as opposed to agricultural) fungicides, e.g. in the prevention of fungal attack on wood, hides, leather and especially paint films.

The compounds may be used as such for fungicidal purposes but are more conveniently formulated into compositions for such usage. The invention thus provides a fungicidal composition comprising a compound of general formula (I) as hereinbefore defined, or a salt, thereof; and, optionally, a carrier or diluent.

The invention also provides a method of combating fungi, which comprises applying to a plant, to seed of a plant, or to the locus of the plant or seed, a compound, or salt, thereof, as hereinbefore defined.

The compounds and their salts can be applied in a number of ways, for example they can be applied, formulated or unformulated, directly to the foliage of a plant, or they can be applied also to bushes and trees, to seeds or to other medium in which plants, bushes or trees are growing or are to be planted, or they can be sprayed on, dusted on or applied as a cream or paste formulation, or they can be applied as a vapour; or as slow release granules. Application can be to any part of the plant, bush or tree, for example to the foliage, stems, branches or roots, or to soil surrounding the roots, or to the seed before it is planted; or to the soil generally, to paddy water or to hydroponic culture systems. The invention compounds may also be injected into plants or trees and they may also be sprayed onto vegetation using electrodynamic spraying techniques.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (eg. 2-ethoxyethanol and 2-butoxyethanol).

The compositions to be used as sprays may also be in the form of aerosols wherein the formulation is held in a container under pressure in the presence of a propellant, eg. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a micro-encapsulated form. They may also be formulated in biodegradable polymeric formulations to obtain a slow, controlled release of the active substance.

By including suitable additives, for example additives for improving the distribution, adhesive power and resistance to rain on treated surfaces, the different compositions can be better adapted for various utilities.

The compounds can be used as mixtures with fertilisers (eg. nitrogen-, potassium-or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising the compound of general formula (I) or a salt thereof.

The compositions may also be in the form of liquid preparations for use as dips or sprays which are generally aqueous dispersions or emulsions containing the active ingredient in the presence of one or more surfactants eg. wetting agent(s), dispersing agent(s), emulsifying agent(s) or suspending agent(s); or which are spray formulations of the kind suitable for use in electrodynamic spraying techniques. The foregoing agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium diisopropyl-and triisopropylnaphthalene sulphonates).

Suitable non-ionic agents are the condensation products of ethylene oxide with fatty alcohols such as oleyl or cetyl alcohol, or with alkyl phenols such as octyl-or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), and the concentrate is to be diluted with water before use. These concentrates often should be able to withstand storage for prolonged periods and after such storage be capable of dilution with water in order to form aqueous preparations which remain homogeneous for a sufficient time to enable them to be applied by conventional and electrodynamic spray equipment. The concentrates may conveniently contain up to 95%, suitably 10-85%, for example 25-60%, by weight of the active ingredient(s). These concentrates suitably contain organic acids (eg. alkaryl or aryl sulphonic acids such as xylenesulphonic acid or dodecyl benzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, eg. compounds having similar or complementary fungicidal activity or compounds having plant growth regulating, herbicidal or insecticidal activity.

The other fungicidal compound can be, for example, one which is capable of combating ear diseases of cereals (eg. wheat) such as Septoria, Gibberella and Helminthosporium spp., seed and soil borne diseases and downy and powdery mildews on grapes and powdery mildew and scab on apple etc. These mixtures of fungicides can have a broader spectrum of activity than the compound of general formula (I) alone; further the other fungicide can have a synergistic effect on the fungicidal activity of the compound of general formula (I). Examples of the other fungicidal compound are carbendazim, benomyl, thiophanate-methyl, thiabendazole, fuberidazole, etridazole, dichlofluanid, cymoxanil, oxadixyl, ofurace, metalaxyl, furalaxyl, benalaxyl, fosetylaluminium, fenarimol, iprodione, procymidione, vinclozolin, penconazole, myclobutanil, R0151297, pyrazophos, ethirimol, ditalimfos, tridemorph, triforine, nuarimol, triazbutyl, guazatine, triacetate salt of 1,1'iminodi(octamethylene)diguanidine, propiconazole, prochloraz, flutriafol, hexaconazole ie. the chemical 1-(1,2,4-triazol-1-yl)-2-(2,4-dichlorophenyl)hexan-2-ol, (2RS, 3RS)-2-(4-chlorophenyl)-3-cyclopropyl-1-(1H,1,2,4-triazol-1-yl)butan-2-ol, (RS)-1-(4-chlorophenyl)-4,4,4-dimethyl-3-(1H-1,2,4-triazol-1-ylmethyl)-pentan-3-ol, DPX H6573(1-((bis-4-fluorophenyl)methylsilyl)methyl)1H-1,2,4-triazole, triadimefon, triadimenol, diclobutrazol, fenpropimorph, fenpropidin, chlorozolinate, diniconazol, imazalil, fenfuram, carboxin, oxycarboxin, methfuroxam, dodemorph, BAS 454, blasticidin S, kasugamycin, edifenphos, kitazin P, cycloheximide, phthalide, probenazole, isoprothiolane, tricyclazole, pyroquilon, 4-chloro-N-(cyano(ethoxy)-methylbenzamide, chlorbenzthiazone, neoasozin, polyoxin D, validamycin A, mepronil, flutolanil, pencycuron, diclomezine, phenazin oxide, nickel dimethyldithiocarbamate, techlofthalam, bitertanol, bupirimate, etaconazole, streptomycin, cypofuram, biloxazol, quinomethionate, dimethirimol, 1-(2-cyano-2-methoxyiminoacetyl)-3-ethyl urea, fenapanil, tolclofos-methyl, pyroxyfur, polyram, maneb, mancozeb, captafol, chlorothalonil, anilazine, thiram, captan, folpet, zineb, propineb, sulphur, dinocap, binapactryl, nitrothalisopropyl, dodine, dithianon, fentin hydroxide, fentin acetate, tecnazene, quintozene, dichloran, copper containing compounds such as copper oxychloride, copper sulphate and Bordeaux mixture, and organomercury compounds.

The compounds of general formula (I) can be mixed with soil, peat or other rooting media for the protection of plants against seed-borne, soil-borne or foliar fungal diseases.

Suitable insecticides which may be incorporated in the composition of the invention include pirimicarb, dimethoate, demeton-s-methyl, formothion, carbaryl, isoprocarb, XMC, BPMC, carbofuran, carbosulfan, diazinon, fenthion, fenitrothion, phenthoate, chlorpyrifos, isoxathion, propaphos, monocrotophos, buprofezin, ethroproxyfen and cycloprothrin.

Plant growth regulating compounds for use in the invention compositions are compounds which control weeds or seedhead formation, or selectively control the growth of less desirable plants (e.g. grasses).

Examples of suitable plant growth regulating compounds for use with the invention compositions are the gibberellins (e.g. $GA_3$, $GA_4$ or $GA_7$), the auxins (e.g. indoleacetic acid, indolebutyric acid, naphthoxyacetic acid or naphthylacetic acid), the cytokinins (e.g. kinetin, diphenylurea, benzimidazole, benzyladenine or benzylaminopurine), phenoxyacetic acids (e.g. 2,4-D or MCPA), substituted benzoic acids (e.g. triiodobenzoic acid), morphactins (e.g. chlorfluoroecol), maleic hydrazide, glyphosate, glyphosine, long chain fatty alcohols and acids, dikegulac, paclobutrazol, flurprimidol, fluoridamid, mefluidide, substituted quaternary ammonium and phosphonium compounds (e.g. chloromequat chlorphonium or mepiquatchloride), ethephon, carbetamide, methyl-3,6-dichloroanisate, daminozide, asulam, abscisic acid, isopyrimil, 1-(4-chlorophenyl)-4,6-dimethyl-2-oxo-1,2-dihydropyridine-3-carboxylic acid, hydroxybenzonitriles (e.g. bromoxynil), difenzoquat, benzoylprop-ethyl 3,6-dichloropicolinic acid, fenpentezol, inabenfide, triapenthenol and tecnazene.

The following Examples illustrate the invention; the temperatures are given in degrees Centigrade (°C).

## EXAMPLE 1

This Example illustrates the preparation of 4-[3-(p-t-butylphenyl)-cis-cyclobutylmethyl]-2,6-cis-dimethylmorpholine (Compound No.3.1 in Table 1). A mixture of p-t-butylbenzyl bromide (20.44 g, 0.09 mol) and potassium cyanide (11.72 g, 0.18 ml) in a saturated solution of 18-crown-6 (50 ml) was refluxed at 80°C for 5 hours. After cooling to room temperature the solution was poured into water, extracted with dichloromethane and dried over anhydrous sodium sulphate. Removal of the solvent gave a yellow liquid which was purified by column chromatography (silica eluted with petroleum ether/ethyl acetate 9:1) to give p-t-butylbenzyl cyanide (15.4 g, 99%).

A solution of p-t-butylbenzyl cyanide (28.6 g, 0.166 mol) and concentrated sulphuric acid (60.7 g) in dry ethanol (60.7 g) was refluxed at 100°C for 7 hour. The mixture was poured into water, extracted with diethyl ether, and dried over anhydrous sodium sulphate. Removal of the solvent gave ethyl p-t-butylphenylacetate (32.3 g, 89%) as a colourless liquid.

A solution of ethyl p-t-butylphenylacetate (32.29 g, 0.147 mol), diethyl carbonate (7.1 g, 0.309 mol) and sodium ethoxide (21 .g 0.309 mol) in toluene (140 ml) was refluxed in a Dean-Stark apparatus for 6 hours. The toluene was removed in vacuo; the resulting oil poured into water, extracted with diethyl ether and dried over anhydrous sodium sulphate. Removal of the solvent gave a brown oil which was purified by column chromatography (silica eluted with petroleum ether/ethyl acetate 4:1) to give diethyl p-t-butylphenylmalonate (29.0 g, 68%) as a colourless liquid.

A solution of diethyl p-t -butylphenylmalonate (27.6 g, 0.095 mol) in sodium dried diethyl ether (300 ml) was added dropwise to a suspension of lithium aluminium hydride (8.3 g, 0.218 mol) in sodium dried ether (300 ml) at such a rate so as to maintain gentle reflux. After the addition the solution was stirred at room temperature for 6 hours, passed carefully into water, acidified with 2M hydrochloric acid, and extracted with diethyl ether. The ethereal extracts were washed with water, dried over anhydrous sodium sulphate and the solvent removed to give 2-(p-t-butylphenyl)propan-1,3-diol (18.8 g, 95%) as a yellow solid which was used in the next stage without further purification.

To a solution of 2-(p-t-butylphenyl)propan-1,3-diol (18.84 g, 0.09 mol) in dry pyridine (100 ml) was added, below 10°, p-toluenesulphonyl chloride (38.5 g, 0.202 mol). After 12 hours at 20°C the mixture was treated with cold hydrochloric acid, and the resulting solid filtered off.

Recrystallisation from ethanol gave 2-(p-t-butylphenyl)propan-1,3-diol-di-p-toluenesulphonate (33.8 g, 73%) as a white crystalline solid mp 135-136°C.

A suspension of sodium hydride (3.1 g, 0.065 mol; 50% suspension in mineral oil) in dry dioxane (40 ml) was added dropwise over 1 hour to a solution of 2-(p-t -butylphenyl)propan-1,3-diol-di-p-toluenesulphonate (33.8 g, 0.0654 mol) and diethyl malonate (11.41 g, 0.0712 mol) in dry dioxane (150 ml) and warmed to almost reflux under nitrogen at such a rate to cause spontaneous refluxing. After stirring and refluxing for 1 hour, a further portion of sodium hydride (31.g, 0.065 mol; 50% suspension in mineral oil) in dry dioxane (40 ml) was added with care over 3 hours. The mixture was refluxed for 10 hours, and the dioxane removed under reduced pressure. The residue was treated with water, extracted with diethyl ether, and the ethereal extracts dried over anhydrous sodium sulphate. Removal of the solvent gave a brown gum which was purified by column chromatography (silica eluted with petroleum ether/ethyl acetate 4:1) to give diethyl 3-(p-t-butylphenyl)cyclobutane-1,1-dicarboxylate (12.9 g, 60%) as a colourless oil.

A mixture of diethyl 3-(p-t-butylphenyl)cyclobutane-1,1-dicarboxylate (8.25 g, 0.025 mol), lithium chloride (2.3g, 0.054 mol), water (30 ml, 1.67 mol) and dimethylsulphoxide (50 ml) was heated at 140-150°C for 12 hours. The mixture was cooled, poured into water, extracted with ethyl acetate, washed with water and dried over anhydrous sodium sulphate. Removal of the solvent gave ethyl 3-(p-t-butylphenyl)cyclobutane-1-carboxylate (4.55 g, 70%) after purification by column chromatography (silica eluted with petroleum ether/ethyl acetate 4:1).

Ethyl 3-(p-t-butylphenyl)cyclobutane-1-carboxylate (4.5 g, 0.017 mol) was dissolved in methanol (40 ml) and a solution of potassium hydroxide (1.9 g, 0.034 mol) in water (40 ml) added dropwise at room temperature. The solution was refluxed for 3 hours, cooled to room temperature and carefully neutralized with 2 M hydrochloric acid. After extraction with ethyl acetate the extracts were washed with water and dried over anhydrous sodium sulphate. Removal of the solvent gave 3-(p-t-butylphenyl)cyclobutane-1-carboxylic acid (3.84 g, 97%) as a yellow solid.

A mixture of 3-(p-t-butylphenyl)cyclobutane-1-carboxylic acid (3.84 g, 0.0166 mol) and oxalyl chloride (3 g, 0.024 mol) in hexane (30 ml) was stirred at room temperature for 1 hour and warmed at 60°C for 3 hours. The hexane and excess oxalyl chloride were removed in vacuo to give 3-(p-t -butylphenyl)-cyclobutane-1-carbonyl chloride (4.14 g, 100%) as a pale yellow liquid which was used in the next stage without further purification.

2,6-Dimethylmorpholine (4.6 g, 0.04 mol) was added dropwise to a solution of 3-(p-t-butylphenyl)-cyclobutane-1-carbonyl chloride (3.0g, 0.012 mol) in sodium, dried diethyl ether (20 mol) at 10°C and after complete addition the solution was stirred at 20°C for 3 hours. The reaction mixture was poured into water and extracted with diethyl ether (2 × 50 ml). The ethereal extracts were washed with water, dried over anhydrous sodium sulphate, and the solvent removed to give 3-(p-t-butylphenyl)cyclobutane-1-carbonyl-2,6-dimethylmorpholine (3.88 g, 98%).

A solution of 3-(p-t-butylphenyl)cyclobutane-1-carbonyl-2,6-dimethylmorpholine (3.63, 0.011 mol) in sodium dried ether (50 ml) was added dropwise to a suspension of lithium aluminium hydride(0.8 g, 0.021 mol) in sodium dried ether (25 ml) and the mixture stirred at room temperature for 3 hours. The reaction mixture was poured carefully into water and extracted with diethyl ether. The ethereal extracts were washed with water, dried over anhydrous sodium sulphate and the solvent removed to give a colourless oil.

This oil was purified by column chromatography (silica gel eluted with petroleum ether/ethyl acetate 9:1) to give the title compound (0.9 g, 26%). Three further isomers (compound numbers 1.1, 2.1, and 4.1 in Table 1) were also isolated.

EXAMPLE 2

This Example illustrates the preparation of 4-[trans -3-(4-trimethylsilylphenyl)cyclobutylmethyl]-cis-2,6-dimethylmorpholine (Compound No. 15.1 in Table I). n-Butyllithium (87.5 ml of a 1.6M solution in hexane, 140 mmol) was added dropwise to a stirred solution of 4-bromostyrene (25.0g, 137 mmol) in dry THF (187 ml) under nitrogen at such a rate to keep the temperature below -60°C. After 1½ hours the temperature was allowed to rise to -55°C and trimethylsilyl chloride (17.5 ml, 138 mmol) was added dropwise. After 30 minutes the mixture was allowed to warm to room temperature and ethyl acetate (1 ml) was added. The mixture was then poured onto water. The organic phase was separated, washed with water (×3) and brine, dried ($MgSO_4$) and evaporated in vacuo to give 4-trimethylsilylstyrene (24.1g, 100%) which was used without further purification.

Trichloroacetyl chloride (25 ml, 224 mmol) in dry ether (100 ml) was added over 30 minutes to a mixture of zinc powder (19.5g, 300 mmol) and 4-trimethylsilylstyrene (25.8g, 147 mmol) in dry ether (300 ml) in a sonic bath. When TLC showed no starting material remained (about 1 hour) the mixture was filtered through celite. The filtrate was washed with water, saturated sodium bicarbonate solution and water, dried ($MgSO_4$) and evaporated in vacuo. Chromatography [$SiO_2$, hexane-ethyl acetate (95:5)] gave 2,2-dichloro-3-(4-trimethylsilylphenyl)cyclobutanone (6.1g, 15%), mp. 65-69°C.

2,2-Dichloro-3-(4-trimethylsilylphenyl)cyclobutanone (6.1g, 21 mmol) and azobisisobutyronitrile (0.1g) in cyclohexane (15 ml) were added to a refluxing solution of tributyltin hydride (12.5 ml, 47 mmol) in cyclohexane (15 ml) under nitrogen. After 3 hours the mixture was cooled, evaporated in vacuo and chromatographed [$SiO_2$, hexane-ethyl acetate (100:0) to (80:20)] to give 3-(4-trimethylsilylphenyl)-cyclobutanone (3.30g, 71%), mp. 48°C.

Dimethyl sulphoxide (10 ml) was added to hexane-washed sodium hydride (0.73g of a 50% dispersion in oil, 16 mmol) under argon. The mixture was heated to 70°C for 1 hour, cooled to 0°C and THF (11 ml) added. Methyltriphenylphosphonium bromide (5.7g, 16 mmol) was added portionwise. After 30 minutes 3-(4-trimethylsilylphenyl)cyclobutanone (3.3g, 15 mmol) was slowly added. After 24 hours the mixture was poured onto water and extracted with hexane. The extracts were washed with water, dried ($MgSO_4$) and evaporated in vacuo. Chromatography ($SiO_2$, hexane) gave 3-(4-trimethylsilylphenyl)-1-methylenecyclobutane (1.33g, 41%).

Borane (2.15 ml of a 1.0 M solution in THF, 2.15 mmol) was added to a stirred solution of 3-(4-trimethylsilylphenyl)-1-methylenecyclobutane (1.33g, 6.2 mmol) in THF (20 ml) at 0°C under nitrogen. After 30 minutes the mixture was warmed to room temperature and 3 M sodium hydroxide solution (0.75 ml, 2.25 mmol) was added followed by hydrogen peroxide solution (0.9 ml 30% w/v, 7.9 mmol) dropwise. The mixture was warmed to 60°C and after 2 hours cooled to room temperature and ether added. The mixture was washed with water and brine, dried ($MgSO_4$), and evaporated in vacuo. Chromatography [$SiO_2$, hexane-ethyl acetate (100:0) to (50:50)] gave 3-(4-trimethylsilylphenyl)cyclobutylmethanol (0.89g, 62%).

Oxalyl chloride (0.83 ml, 9.5 mmol) was added dropwise to a solution of dimethyl sulphoxide (0.83 ml, 11 mmol) in dry dichloromethane (24 ml) at -78°C. After 15 minutes 3-(4-trimethylsilylphenyl)-cyclobutylmethanol (0.89g, 3.8 mmol) in dry dichloromethane (15 ml) was added to the mixture. After 1 hour triethylamine (4.2 ml, 30 mmol) was added and the mixture warmed to room temperature. Hexane (100 ml) and water (100 ml) were added and the organic layer separated, washed with water (×6), dried ($MgSO_4$) and evaporated in vacuo to give 3-(4-trimethylsilylphenyl)cyclobutanecarboxaldehyde (0.82g, 93%).

Hydrogen chloride (3.5 ml of a 2.5 M solution in methanol, 8.7 mmol) was added to a stirred mixture of 3-(4-trimethylsilylphenyl)cyclobutanecarboxaldehyde (0.82g, 3.5 mmol), cis-2,6-dimethyl-morpholine (2.43g, 21 mmol), tetrabutylammonium cyanoboro-hydride (1.50g, 5.3 mmol) and 4Å molecular sieves (1g) in dry dichloromethane (8 ml) under nitrogen. After 16 hours the mixture was filtered (celite), washed with 2M sodium hydroxide solution (×3) and water (×6), dried ($MgSO_4$) and evaporated in vacuo. Chromatography [$SiO_2$, hexane-ethyl acetate (80:20)] gave 4-[trans -3-(4-trimethylsilylphenyl)-cyclobutylmethyl]-cis-2,6-dimethylmorpholine (0.264g, 23%). The cis isomer (Compound No. 14.1 in Table I) was also isolated (0.368g, 32%).

## EXAMPLE 3

This Example illustrates the preparation of 4-[3-(p-i -propylphenyl)cyclopentylmethyl]-2,6-cis-dimethylmorpholine (Compound No. 1.2 in Table II). Succinic anhydride (20 g, 0.2 mol) was stirred in i-propylbenzene (60 g, 0.5 mol) and powdered aluminium chloride (53.3 g, 0.4 mol) added portionwise over a period of 15 minutes. After the addition the resulting suspension was heated at 80° for 10 hours and cooled to room temperature. The dark red oil was poured carefully into ice-water and extracted with ethyl acetate (3 × 200 ml). The ethyl acetate extracts were washed with saturated sodium bicarbonate (3 × 150 ml) and the aqueous extract acidified with concentrated hydrochloric acid. Extraction of the aqueous extract with ethyl acetate followed by drying over anhydrous sodium sulphate and removal of the solvent in vacuo gave an off white solid. Washing this solid with ice cold petroleum ether gave 4-(p-i-propylphenyl)-4-oxo-butanoic acid (30 g, 68%) mp 140 - 141°C as a white crystalline solid.

Thallium (III) nitrate (44.4 g, 0.1 mol), 4-(p-i-propylphenyl)-4-oxo-butanoic acid (22.0 g, 0.1 mol) in trimethyl orthoformate (500 ml) were stirred together at room temperature and perchloric acid (40 g, 0.4 mol) added dropwise. An exothermic reaction took place, the temperature rose to 40°C, and a white precipitate of thallium (I) nitrate was produced. The reaction was stirred a room temperature for 4 hours, the thallium salts filtered off, and the solution extracted with diethyl ether. The ethereal extracts were washed with water and dried over anhydrous sodium sulphate. Removal of the solvent gave a yellow oil which was purified by column chromatography (silica eluted with ethyl acetate/petroleum ether 3:7) to give dimethyl p-i-propylphenylsuccinate (18.0 g, 68%) as a pale yellow oil.

Lithium aluminium hydride (4.6 g, 0.12 mol) was suspended in sodium dried ether (150 ml) and dimethyl p-i-propylphenyl succinate (13,2 g, 0.05 mol) in sodium dried ether (100 ml) was added dropwise at such a rate so as to maintan gentle reflux. The solution was stirred at room temperature for 4 hours, poured carefully into water, acidified with concentrated hydrochloric acid, and extracted with ethyl acetate (3 × 150 ml). The ethyl acetate extracts were washed with water and dried over anhydrous sodium sulphate. Removal of the solvent in vacuo gave 2-(p-i-propylphenyl)butan-1,4-diol (10.0 g, 96%) as a yellow oil.

2-(p-i-Propylphenyl)butan-1,4-diol (4.16 g, 0.02 mol) was stirred in dry pyridine (10 ml) at 5°C and mesyl chloride (4.6 g, 0.04) added dropwise at room temperature. The temperature rose to 30°C and after complete addition the reaction was stirred at room temperature for 3 hours. The reaction mixture was poured into water and extracted with diethyl ether (2 × 100 ml). The ethereal extracts were washed with 2 M hydrochloric acid (2 × 100 ml), water (2 × 100 ml) and dried over anhydrous sodium sulphate. Removal of the solvent gave a white crystalline solid which was triturated with petroleum ether, filtered and dried to give 2-(p-i-propylphenyl)butan-1,4-diol-dimethanesulphonate (7.0 g, 96%), mp 80-81°C.

A suspension of sodium hydride (0.72 g, 0.015 mol; 50% suspension in mineral oil) in dry dioxane (5 ml) was added dropwise over ½ hour to a solution of 2-(p-i-propylphenyl)butan-1,4-diol-dimethanesulphonate (5.22 g, 0.015 mol), diethyl malonate (2.72 g, 0.017 mol) in dry dioxane (30 mol) warmed to almost reflux under nitrogen at such a rate to cause spontaneous refluxing. After stirring and refluxing for 1 hour, a further portion of sodium hydride (0.72 g, 0.015 mol; 50% suspension in mineral oil) in dry dioxane (5 ml) was added with care over 1 hour. The mixture was refluxed for 15 hour and the dioxane removed under reduced pressure. The residue was treated with water, extracted with diethyl ether, and the ethereal extracts dried over anhydrous sodium sulphate. Removal of the solvent gave an orange oil which was purified by column chromatography (silica eluted with petroleum ether/ethyl acetate 9:1) to give diethyl 3-(p-i-propylphenyl)-cyclopentane-1,1-dicarboxylate (3.0 g, 64%) as a colourless liquid.

A mixture of diethyl 3-(p-i-propylphenyl)cyclopentane-1,1-dicarboxylate (2.11 g, 0.00653 mol), lithium chloride (0.54 g, 0.013 mol), water (0.114 g, 0.00635 mol) and dimethyl sulphoxide (11 ml) was heated under reflux for 4 hours. The mixture was cooled, poured into water, extracted with diethyl ether, washed with water and dried over anhydrous sodium sulphate. Removal of the solvent gave ethyl 3-(p-i-propylphenyl)cyclopentane-1-carboxylate (0.92 g, 56%).

Ethyl 3-(p-i-propylphenyl)cyclopentane-1-carboxylate (0.81 g, 0.003 mol) was dissolved in methanol (8 ml) and a solution of potassium hydroxide (0.35 g, 0.006 mol) in water (8 ml) added dropwise at room temperature. The solution was refluxed for 3 hours, cooled to room temperature and carefully neutralised with 2 M hydrochloric acid. After extraction with ethyl acetate the extracts were washed with water and dried over anhydrous sodium sulphate. Removal of the solvent gave 3-(p-i -propylphenyl)cyclopentane-1-carboxylic acid (0.63 g, 87%) as a light brown oil.

A mixture of 3-(p-i-propylphenyl)cyclopentane-1-carboxylic acid (0.52 g, 0.002 mol) and oxalyl chloride (0.4 g, 0.003 mol) in hexane (4 ml) was stirred at room temperature for 1 hour and warmed at 60°C for 3 hours. The hexane and excess oxalyl chloride were removed in vacuo to give 3-(p-i-propylphenyl)-cyclopentane-1-carbonyl chloride (0.55 g, 100%) as a yellow liquid which was used in the next stage without further purification.

cis-2,6-Dimethylmorpholine (0.97 g, 0.0085 mol) was added dropwise to a solution of 3-(p-i -propylphenyl)cyclopentane-1-carbonyl chloride (0.53 g, 0.0021 mol) in sodium dried diethyl ether (4 ml) at 10° and after complete addition the solution was stirred at 20° for 3 hours. The reaction mixture was poured into water and extracted with diethyl ether (2 × 50 ml). The ethereal extracts were washed with water, dried over anhydrous sodium sulphate, and the solvent removed to give 3-(p-i-propylphenyl)-cyclopentane-1-carbonyl-2,6-cis -dimethylmorpholine (0.61 g, 88%).

A solution of 3-(p-i-propylphenyl)cyclopentane-1-carbonyl-2,6-cis-dimethylmorpholine (0.51 g, 0.0016 mol) in sodium dried ether (3 ml) was added dropwise to a suspension of lithium aluminium hydride (0.088 g, 0.0023 mol) in sodium dried ether (3 ml) and the mixture stirred at room temperature for 3 hours. The reaction mixture was poured carefully into water and extracted with diethyl ether. The ethereal extracts were washed with water, dried over anhydrous sodium sulphate, and the solvent removed to give a colourless oil. This oil was purified by column chromatography (silica eluted with petroleum ether/ethyl acetate 7:3) to give the title compound (0.41 g, 84%) as a colourless oil.

## EXAMPLE 4

This Example illustrates the preparation of 4-[3-(p-s-butylphenyl)cyclopentylmethyl]-cis-2,6-dimethyl-morpholine (compound No. 8.2 in Table II). Succinic anhydride (20.0g, 200 mmol) was stirred in s-butylbenzene (87g, 500 mmol) and powdered aluminium chloride (53.3g, 400 mmol) was added portionwise over 5 minutes at room temperature. The mixture was then heated to 80°C and after 10 hours cooled to room temperature. The mixture was poured carefully into ice-water and then extracted with ethyl acetate (×3). The combined extracts were washed wtih saturated sodium bicarbonate solution (×3) and the aqueous fraction acidified with concentrated hydrochloric acid and then extracted with ethyl acetate (×3). The combined organic extracts were washed with water and brine, dried ($Na_2SO_4$) and evaporated in vacuo to give a light brown solid. The solid was washed repeatedly with cold petroleum ether (15 ml) to leave 4-(p-s-butylphenyl)-4-oxo-butanoic acid (13.0g, 28%) as a white crystalline solid.

Perchloric acid (17.17g, 171 mmol) was added dropwise to a stirred mixture of 4-(p-s-butylphenyl)-4-oxo-butanoic acid (10.0g, 42.7 mmol), trimethyl orthoformate (180 ml) and thallium (III) nitrate (18.99g, 42.7 mmol) at room temperature. After 4 hours the mixture was filtered and the filtrate extracted into ether. The extracts were washed with water, dried ($Na_2SO_4$) and evaporated in vacuo. Chromatography [$SiO_2$, petro-leum ether-ethyl acetate (90:10) to (70:30)] gave dimethyl p-s-butylphenylsuccinate (8.82g, 74%).

Dimethyl p-s-butylphenylsuccinate (12.lg, 43.5 mmol) in dry ether (75 ml) was added to a stirred suspension of lithium aluminium hydride (4.01g, 105.6 mmol) in dry ether (75 ml) at such a rate to maintain gentle reflux. After 4 hours at room temperature the mixture was poured carefully into water. The resulting mixture was acidified with concentrated hydrochloric acid and extracted with ether (×3). The combined extracts were washed wtih 2M hydrochloric acid and water (×3), dried ($Na_2SO_4$) and evaporated in vacuo to give 2-(p-s-butylphenyl)butan-1,4-diol (0.51g, 98%).

Mesyl chloride (9.55g, 83.4 mmol) was added slowly to a stirred solution at 2-(p-s-butylphenyl)butan-1,4-diol (9.25g, 41.7 mmol) in dry triethylamine (50 ml) at 5°C. After stirring at room temperature for 10 hours the mixture was poured into water and the resultant mixture extracted with ether (×3). The combined extracts were washed with water, 2M hydrochloric acid (×2) and water, dried ($Na_2SO_4$) and evaporated invacuo to give a brown oil. The oil was triturated with petroleum ether-diethyl ether (90:10) to give 2-(p-s-butylphenyl)butan-1,4-diol-dimethanesulphonate (8.46g, 54%) which was used without further purification.

Sodium hydride (1.06g of a 50% dispersion in oil, 22.1 mmol) was added portionwise to a stirred solution of 2-(p-s-butylphenyl)butan-1,4-diol-dimethanesulphonate (8.35g, 22.1 mmol) and diethyl malonate (4.07g, 25.4 mmol) in dry dioxane (45 ml) at such a rate to maintain gentle reflux. After stirring under reflux for 1 hour sodium hydride (1.06g of a 50% dispersion in oil, 22.1 mmol) was added carefully. After a further 8 hours stirring under reflux the mixture was cooled and evaporated under reduced pressure. Water was added to the residue and the mixture extracted with ether (×2). The combined extracts were washed with water and brine, dried ($Na_2SO_4$) and evaporated in vacuo. Chromatography [$SiO_2$, petroleum ether - ethyl acetate (90:10)] gave diethyl 3-(p-s-butylphenyl)cyclopentane-1,1-dicarboxylate (2.51g, 33%).

Diethyl 3-(p-s-butylphenyl)cyclopentane-1,1-dicarboxylate (2.32g, 13.4 mmol), lithium chloride (0.57g, 13.4 mmol), water (0.121g, 6.7 mmol) and dimethyl sulphoxide (11 ml) were refluxed for 3 hours. The mixture was then poured into water and the aqueous layer saturated with sodium chloride. The mixture was extracted with hexane (×4) and the combined extracts were washed with brine, dried ($Na_2SO_4$) and evaporated in vacuo. Chromatography [$SiO_2$, petroleum ether - ethyl acetate (90:10)] gave ethyl 3-(p-s-butylphenyl)cyclopentane-1-carboxylate (1.28g, 77%).

A solution of potassium hydroxide (0.47g, 8.4 mmol) in water (11 ml) was added to ethyl 3-(p-s-butylphenyl)cyclopentane-1-carboxylate (1.15g, 4.2 mmol) in methanol (11 ml). After stirring for 1 hour the mixture was refluxed for 5 hours, cooled, acidified with 2M hydrochloric acid and extracted with ethyl acetate. The extracts were washed with brine, dried ($MgSO_4$) and evaporated in vacuo to give 3-(p-s-butylphenyl)cyclopentane-1-carboxylic acid (1.08g, 100%).

Oxalyl chloride (1.20g, 9.45 mmol) was added dropwise over 1 minute to a stirred suspension of 3-(p-s-butylphenyl)cyclopentane-1-carboxylic acid (0.93g, 3.78 mmol) in petroleum ether (7 ml). After 1 hour at room temperature the mixture was refluxed for 8 hours, cooled and filtered through celite. The filtrate was evaporated in vacuo to give 3-(p-s-butylphenyl)cyclopentane-1-carbonyl chloride (1.12g, 100%) which was used without further purification.

cis 2,6-Dimethylmorpholine (1.84g, 16 mmol) was added dropwise to a stirred solution of 3-(p-s-butylphenyl)cyclopentane-1-carbonyl chloride (1.06g, 4 mmol) in dry ether (8 ml) at 5°C. After 4 hours at room temperature the mixture was poured into water and extracted with ether. The combined extracts were washed with saturated sodium bicarbonate solution, water and brine, dried ($Na_2SO_4$) and evaporated in vacuo to give 3-(p-s-butylphenyl)cyclopentane-1-carbonyl-2,6-cis-dimethylmorpholine (1.26g, 92%) which was used without further purification.

3-(p-s-Butylphenyl)cyclopentane-1-carbonyl-2,6-cis-dimethylmorpholine (1.05g, 3.06 mmol) in dry ether (5 ml) was added to a stirred suspension of lithium aluminium hydride (0.193g, 5.07 mmol) in dry ether (5 ml) dropwise, so as to maintain steady effervescence. After 2 hours at room temperature the mixture was added carefully to water and the mixture extracted with ether. The combined extracts were washed with water and brine, dried ($Na_2SO_4$) and evaporated in vacuo. Chromatography [$SiO_2$, petroleum ether - ethyl acetate (70:30)] gave 4-[3(p-s-butylphenyl)cyclopentylmethyl]-cis-2,6-dimethylmorpholine (0.91g, 90%) as a colourless oil.

EXAMPLE 5

This Example illustrates the preparation of 4-[3-(4-chlorophenyl)cyclopentylmethyl]-2,6-cis-dimethylmorpholine (compound No. 14.2 of Table 2). n-Butyllithium (26 ml of a 1.55 M solution in hexane, 40.3 mmol) was added dropwise to a stirred solution of 4-bromochlorobenzene (7.66g, 40 mmol) in dry ether (60 ml) at 0°C under nitrogen. After 1 hour at room temperature the mixture was added to anhydrous zinc bromide (4.5g, 20 mmol) in dry ether (20 ml) and the resulting mixture placed in a sonic bath for 30

minutes when all the zinc bromide had dissolved. 2-Cyclopentenone (0.82g, 10 mmol) and nickel acetylacetonate (0.06g, 0.2 mmol) in ether (10 ml) were added to the stirred mixture. After 1 hour at room temperature the mixture was added to saturated aqueous ammonium choride solution and the mixture extracted with ether. The combined extracts were dried ($MgSO_4$) and evaporated in vacuo. Chromatography [$SiO_2$, hexane-ethyl acetate (80:20)] gave 3-(4-chlorophenyl)cyclopentanone) (1.03g, 53%).

Dimethyl sulphoxide (5 ml) was added to hexane-washed sodium hydride (0.461g of a 55% suspension in oil, 10.6 mmol) under nitrogen and the mixture stirred at 70°C for 1 hour. The mixture was cooled to 0°C and THF (10 ml) added followed by methyltriphenylphosphonium bromide (3.786g, 10.6 mmol). After stirring for 1 hour 3-(4-chlorophenyl)cyclopentanone) (1.03g, 5.3 mmol) in THF (5 ml) was added. After 3 hours at 0°C hexane was added and the mixture filtered through celite. The filtrate was evaporated in vacuo and chromatographed ($SiO_2$, hexane) to give 3-(4-chlorophenyl)-1-methylenecyclopentane (0.56g, 55%).

Borane (1.0 ml of a 1M solution in THF, 1 mmol) was added to a stirred solution of 3-(4-chlorophenyl)-1-methylenecyclopentane (0.56g, 2.9 mmol) in THF (10 ml) at 0°C under nitrogen. After 30 minutes the mixture was allowed to warm to room temperature and 3M aqueous sodium hydroxide (0.34 mls, 1.02 mmol) followed by hydrogen peroxide (0.43 ml 30% aqueous w/v, 3.8 mmol) were added dropwise. The mixture was heated to 60°C and after 30 minutes allowed to cool to room temperature. The mixture was extracted with ether and the combined extracts washed with water and brine, dried ($MgSO_4$) and evaporated in vacuo. Chromatography [$SiO_2$, hexane-ethyl acetate (50:50)] gave 3-(4-chlorophenyl)-1-(hydroxymethyl)-cyclopentane (0.342g, 53%).

Dimethyl sulphoxide (0.35 ml, 4.9 mmol) was added to dry dichloromethane (10 ml) under nitrogen, the mixture cooled to -78°C and oxalyl chloride (0.35 ml, 4.0 mmol) was added dropwise. After 15 minutes 3-(4-chlorophenyl)-1-(hydroxymethyl)cyclopentane (0.342g, 1.6 mmol) in dry dichloromethane (5 ml) was added. After 1 hour triethylamine (1.78 ml, 128 mmol) was added and the mixture allowed to warm to room temperature. Hexane and water were added and the mixutre filtered through celite. The organic layer was separated and washed with water (×6), dried and evaporated invacuo to give 3-(4-chlorophenyl)-cyclopentanecarboxaldehyde (0.326g, 98%) which was used without further purification.

Hydrogen chloride in methanol (3.5 ml of a 2.6 M solution, 9.1 mmol) was added to a stirred mixture of 3-(4-chlorophenyl)cyclopentanecarboxaldehyde (0.326g, 1.56 mmol), tetrabutylammonium cyanoborohydride (0.884g, 3.1 mmol), cis 2,6-dimethylmorpholine (2.21g, 19.2 mmol) and 4Å molecular sieves (1.5g) in dry dichloromethane (20 ml). After 17 hours the reaction mixture was filtered and the filtrate washed with 2M sodium hydroxide solution (×3). The aqueous fractions were extracted with ether and the combined organic fractions washed with water (×4), dried ($MgSO_4$) and evaporated in vacuo. Chromatography [$SiO_2$, hexane-ethyl acetate (70:30)] gave 4-[3-(4-chlorophenyl)cyclopentylmethyl]-2,6-cis-dimethylmorpholine (0.262g, 55%), as a 50:50 mixture of cis and trans isomers.

## EXAMPLE 6

This Example illustrates the preparation of 4-[trans-2-(4-t-butylphenyl)cyclobutylmethyl]-2,6-cis-dimethylmorpholine (Compound No. 1.3 Table III). Magnesium (4.86g, 200 mmol) was added to a stirred solution of methyl p-t-butylcinnamate (10.5g, 48.1 mmol) in dry methanol (500 ml) and the mixture warmed to 40°C to initiate the reaction. Once initiated the reaction was maintained at approximately 20°C for 2 hours after which the reaction was complete. Hexane (100 ml) and ether (10 ml) were added followed by 2M hydrochloric acid to dissolve the precipitate. The organic layer was separated and washed with water (×6), dried ($Na_2SO_4$), evaporated in vacuo and filtered through silica gel to give methyl 3-(4-t-butylphenyl)-propionate (9.0g, 82%).

Di-isobutylaluminium hydride (30.7 ml of an approximately 2M solution in toluene, approximately 46.0mmol) was added dropwise over 10 minutes to a stirred solution of methyl 3-(4-t-butylphenyl)propionate (9.0g, 40.9 mmol) in dry toluene (100 ml) at -78°C. After 2½ hours methanol (10 ml) was added and the mixture allowed to warm to room temperature. 2M Hydrochloric acid was added to dissolve the precipitate and the organic layer separated and washed with water (×5), dried ($Na_2SO_4$) and evaporated in vacuo. Chromatography [$SiO_2$, t -butyl methyl ether-hexane (10:90) to (40:60)] gave 3-(4-t -butylphenyl)propan-1-ol (4.1g, 52%).

Triphenylphosphine (11.23g, 85.2 mmol) was added to a solution of 3-(4-t-butylphenyl)propan-1-ol (4.1g, 21.3 mmol) in 1,2-dichloroethane (18 ml) and the mixture cooled to -15°C. 1,2-Dibromotetrachloroethane (13.94g, 42.6 mmol) in 1,2-dichloroethane (24 ml) and triethylamine (8.65 ml, 42.6 mmol) were added simultaneously over 10 minutes. After 20 minutes the mixture was allowed to warm to room temperature, evaporated in vacuo , dissolved in dichloromethane, washed with water (×3) and brine and evaporated in vacuo. The residue was dissolved in ether, filtered through celite, evaporated in vacuo and chromatographed [$SiO_2$, t-butyl methyl ether-hexane (1:99)] to give 1-bromo-3-(4-t-butylphenyl)propane (4.60, 85%)

1-Bromo-3-(4-t-butylphenyl)propane (4.51g, 17.7 mmol), N-bromosuccinimide (3.15g, 17.7 mmol) and azobisisobutyronitrile (100 mg) were refluxed in carbon tetrachloride (40 ml) with irradiation from a 60w bulb for 2 hours. The mixture was then cooled, filtered through celite, evaporated in vacuo and chromatographed ($SiO_2$, hexane) to give 1,3-dibromo-1-(4-t-butylphenyl)propane (3.0g, 51%).

1,3-Dibromo-1-(4-t -butylphenyl)propane (3.0g, 9.0 mmol) and diethyl malonate (1.52g, 9.5 mmol) were dissolved in dry dioxane (20 ml) and heated to 90°C. Hexane-washed sodium hydride (0.432g of a 50% dispersion in oil, 9.0 mmol) in dry dioxane (3 ml) was added dropwise over 5 minutes so as to maintain reflux. The mixture was then refluxed for 1 hour. Hexane-washed sodium hydride (0.432g of a 50% dispersion in oil, 9.0 mmol) in dry dioxane (4 ml) was added dropwise over 5 minutes. The mixture was then refluxed for 4 hours, cooled to room temperature, ether added and the mixture washed with water (×4) and brine, dried ($Na_2SO_4$) and evaporated in vacuo. Chromatography [$SiO_2$, t-butyl methyl ether-hexane (10:90)] gave diethyl 2-(4-t-butylphenyl)cyclobutane-1,1-dicarboxylate (1.931g, 58%).

Diethyl 2-(4-t-butylphenyl)cyclobutane-1,1-dicarboxylate (1.90g, 5.73 mmol), lithium chloride (0.51g, 12 mmol) and water (0.11 ml, 6 mmol) were added to dimethyl sulphoxide (10 ml) and the mixture refluxed for 4 hours. The mixture was allowed to cool to room temperature, hexane (50 ml) and ether (50 ml) added, and the mixture washed with brine, water (×5) and brine, dried ($Na_2SO_4$) and evaporated in vacuo to give ethyl 2-(4-t-butylphenyl)cyclobutanecarboxylate (0.82g, 55%) which was used without further purification.

Ethyl 2-(4-t-butylphenyl)cyclobutanecarboxylate (0.80g, 3.08 mmol) was stirred in 2M sodium ethoxide (8 ml) for 6 hours under argon. Water (2 ml) was then added and the mixture stirred for 90 hours at room temperature. Ether was then added, the mixture washed with 2M hydrochloric acid and water (×3), dried ($Na_2SO_4$) and evaporated in vacuo to give trans 2-(4-t-butylphenyl)cyclobutanecarboxylic acid (0.710g, 100%).

Oxalyl chloride (0.33 ml, 3.78 mmol) was added to trans 2-(4-t -butylphenyl)cyclobutanecarboxylic acid (0.71g, 3.08 mmol) in hexane (5 ml) and the mixture refluxed for 2 hours. The mixture was then cooled, evaporated in vacuo and ether (5 ml) added. The mixture was then cooled to 0°C and cis 2,6-dimethylmorpholine (1.40g, 12.3 mmol) added. After 15 minutes ether was added and the mixture washed with water (×3), dried ($Na_2SO_4$) and evaporated in vacuo. Chromatography [$SiO_2$, t-butyl methyl ether-hexane (25:75) to (50:50)] gave trans 2-(4-t-butylphenyl)cyclobutanecarbonyl-2,6-cis-dimethylmorpholine (0.71g, 70%).

trans 2-(4-t-Butylphenyl)cyclobutanecarbonyl-2,6-cis-dimethylmorpholine (0.69 2.10 mmol) in dry THF (5 ml) was added to a stirred suspension of lithium aluminium hydride (0.160g, 4.2 mmol) in dry THF (15 ml) at 0°C under argon. The mixture was stirred at room temperature for 4 hours. Ethyl acetate (1 ml) was then added at 0°C followed by ethanol (2 ml) and water (5 ml). The mixture was partitioned between ether and aqueous sodium potassium tartrate solution and the organic layer separated, washed with water (×2) and brine, dried ($Na_2SO_4$) and evaporated in vacuo. Chromatography [$SiO_2$, t-butyl methyl ether-hexane (30:70)] gave 4-[trans-2-(4-t-butylphenyl)cyclobutylmethyl]-2,6-cis-dimethylmorpholine (0.56g, 85%).

EXAMPLE 7

An emulsifiable concentrate was made up by mixing the ingredients, and stirring the mixture until all the constituents were dissolved.

Compound of Example 4 10%
Ethylene dichloride 40%
Calcium dodecylbenzenesulphate 5%
"Lubrol" L 10%
"Aromasol" H 35%

EXAMPLE 8

A composition in the form of grains readily dispersible in a liquid, e.g. water, was prepared by grinding together the first three ingredients in the presence of added water and then mixing in the sodium acetate. The resultant mixture was dried and passed through a British Standard mesh sieve, size 44-100, to obtain the desired size of grains.

Compound of Example 4 50%
"Dispersol" T 25%
"Lubrol" APN5 1.5%
Sodium acetate 23.5%

EXAMPLE 9

The ingredients were all ground together to produce a powder formulation readily dispersible in liquids.

Compound of Example 4 45%
"Dispersol" T 5%
"Lissapol" NX 0.5%
"Cellofas" B600 2%
Sodium acetate 47.5%

EXAMPLE 10

The active ingredient was dissolved in a solvent and the resultant liquid was sprayed on to the granules of China clay. The solvent was then allowed to evaporate to produce a granular composition.

Compound of Example 4 5%
China clay granules 95%

EXAMPLE 11

A composition suitable for use as a seed dressing was prepared by mixing the three ingredients.

Compound of Example 4 50%
Mineral oil 2%
China clay 48%

EXAMPLE 12

A dusting powder was prepared by mixing the active ingredient with talc.

Compound of Example 4 5%
Talc 95%

EXAMPLE 13

A Col formulation was prepared by ball-milling the constituents set out below and then forming an aqueous suspension of the ground mixture with water.
Compound of Example 4 40%
"Dispersol" T 10%
"Lubrol" APN5 1%
Water

EXAMPLE 14

A dispersible powder formulation was made by mixing together the ingredients set out below and then grinding the mixture until all were thoroughly mixed.
Compound of Example 4 25%
"Aerosol" OT/B 2%
"Dispersol" A.C. 5%
China clay 28%
Silica 40%

EXAMPLE 15

This Example illustrates the preparation of a dispersible powder formulation. The ingredients were mixed and the mixture then ground in a comminution mill.
Compound of Example 4 25%
"Perminal" BX 1%
"Dispersol" T 5%
Polyvinylpyrrolidone 10%
Silica 25%
China clay 34%

EXAMPLE 16

The ingredients set out below were formulated into a dispersible powder by mixing then grinding the ingredients.
Compound of Example 2 25%
"Aerosol" OT/B 2%
"Dispersol" A 5%
China clay 68%

In Examples 7 to 16 the proportions of the ingredients given are by weight.

The remaining compounds set out in Tables I, II and III were similarly formulated as specifically described in Examples 7 to 16.

There now follows an explanation of the compositions or substances represented by the various Trade Marks mentioned above.

LUBROL L : a condensate of nonyl phenol 1 mole) with ethylene oxide (13 moles)

AROMASOL H : a solvent mixture of alkylbenzenes

DISPERSOL T & AC : a mixture of sodium sulphate and a condensate of formaldehyde with sodium naphthalene sulphonate

LUBROL APN5 : a condensate of nonyl phenol (1 mole) with naphthalene oxide (5.5 moles)

CELLOFAS B600 : a sodium carboxymethyl cellulose thickener

LISSAPOL NX : a condensate of nonyl phenol (1 mole) with ethylene oxide (8 moles)

AEROSOL OT/B : dioctyl sodium sulphosuccinate

PERMINAL BX : a sodium alkyl naphthalene sulphonate

EXAMPLE 17

The compounds were tested against a variety of foliar fungal diseases of plants. The technique employed was as follows.

The plants were grown in John Innes Potting Compost (No 1 or 2) in 4 cm diameter minipots. The test compounds were formulated either by bead milling with aqueous Dispersol T or as a solution in acetone or acetone/ethanol which was diluted to the required concentration immediately before use. For the foliar diseases, the formulations (100 ppm active ingredient) were sprayed on to the foliage and applied to the roots of the plants in the soil. The sprays were applied to maximum retention and the root drenches to a final concentration equivalent to approximately 40 ppm a.i./dry soil. Tween 20, to give a final concentration of 0.05%, was added when the sprays were applied to cereals.

For most of the tests the compound was applied to the soil (roots) and to the foliage (by spraying) one or two days before the plant was inoculated with the disease. An exception was the test on Erysiphe graminis in which the plants were inoculated 24 hours before treatment. Foliar pathogens were applied by spray as spore suspensions onto the leaves of test plants. After inoculation, the plants were put into an appropriate environment to allow infection to proceed and then incubated until the disease was ready for assessment. The period between inoculation and assessment varied from four to fourteen days according to the disease and environment.

The disease control was recorded by the following grading:-

4 = no disease
3 = trace - 5% of disease on untreated plants
2 = 6-25% of disease on untreated plants
1 = 26-59% of disease on untreated plants
0 = 60-100% of disease on untreated plants

The results are shown in Table VIII.

TABLE VIII

| COMPOUND NUMBER | PUCCINIA RECONDITA (WHEAT) | ERYSIPHE GRAMINIS (BARLEY) | VENTURIA INAEQUALIS (APPLE) | CERCOSPORA ARACHIDICOLA (PEANUT) |
|---|---|---|---|---|
| 1.1 | 0 | 4 | 0 | 0 |
| 2.1 | 0 | 4 | 0 | 0 |
| 3.1 | 1 | 4 | 4 | 0 |
| 4.1 | 1 | 4 | 3 | 3 |
| 5.1 | 0 | 4 | 1* | 3 |
| 6.1 | 0 | 2 | 0 | 0 |
| 7.1 | 0 | 4 | 0 | 3 |
| 8.1 | 0 | 4 | 0 | 0 |
| 9.1 | 0 | 4 | 0 | 0 |
| 1.2 | 0 | 4 | 4 | 4 |
| 3.2 | 0 | 4 | 4 | 2 |
| 5.2 | 0 | 4 | 0 | 2 |
| 6.2 | 0 | 4 | 0 | 0 |
| 7.2 | 0 | 4 | 0 | 0 |
| 8.2 | 0 | 4 | 0 | 1 |
| 1.3 | 0 | 4 | 4 | 3 |

* Foliar spray only at 100 ppm.

## Claims

1. Compounds having the general formula:-

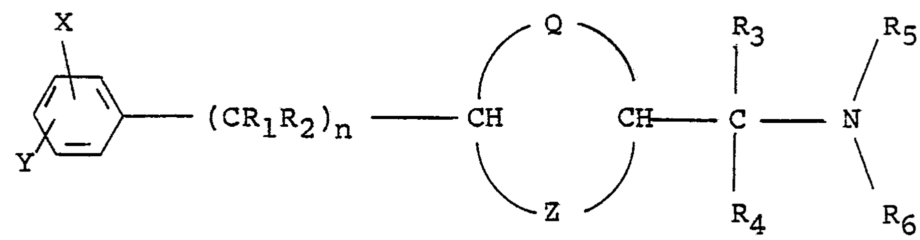

(I)

and stereoisomers thereof, wherein $R_1$ and $R_2$ each represent a hydrogen atom, a halogen atom, or an alkyl group containing from 1 to 4 carbon atoms, $R_3$ and $R_4$ each represent a hydrogen atom or an alkyl group containing from 1 to 4 carbon atoms; $R_5$ and $R_6$ each represent an alkyl group containing from 1 to 4 carbon atoms or $R_5$ and $R_6$ together with the adjacent nitrogen atom form a heterocyclic ring which may optionally contain an additional hetero atom, X and Y each represent a hydrogen atom, a halogen atom, or an alkyl, cycloalkyl, cycloalkylalkyl, alkenyl, alkynyl, aryl, aralkyl, alkoxy or aryloxy group, or a

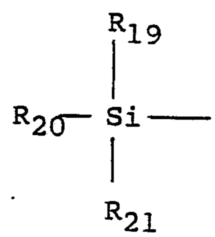

group wherein $R_{19}$, $R_{20}$ and $R_{21}$ are each alkyl, alkenyl, alkynyl, cycloalkyl, cycloalkylalkyl, aryl or aralkyl; n has the value 0 or 1 and Q is a bridging bridging group having one of the following values $Q_1$, $Q_2$, $Q_3$ or $Q_4$.

$Q_1$ =

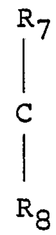

$Q_2$ =

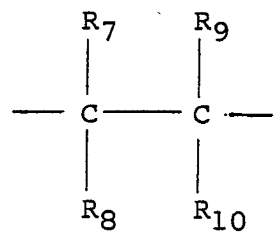

$Q_3$ =

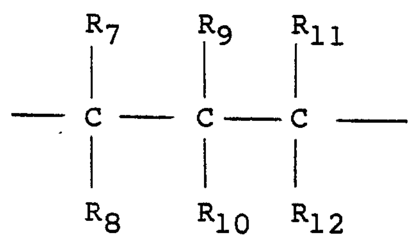

$Q_4$ =

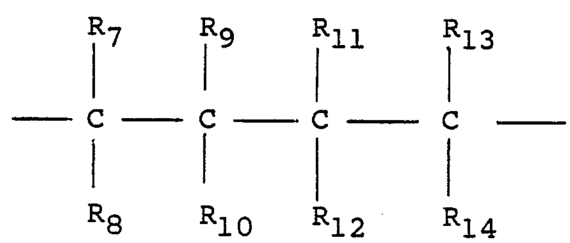

wherein $R^7$ to $R^{14}$ each independently represent a hydrogen atom, a halogen atom, a hydroxyl group or an alkyl group containing from 1 to 4 carbon atoms; Z is a bridging group which is a covalent linkage, denoted by $Z_o$, or has one of the following values $Z_1$ or $Z_2$:

$Z_1$ =

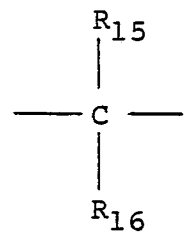

$Z_2$ =

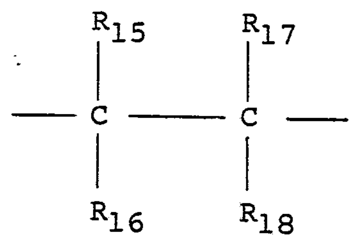

wherein $R_{15}$ to $R_{18}$ each independently has the same significance as $R_7$ to $R_{14}$ above; provided that

(i) when Z has the value $Z_o$, Q may not have the value $Q_1$;

(ii) the total number of carbon atoms forming part of the ring substance in formula (I) and contributed by the groups Q and Z does not exceed 4; and acid addition salts thereof.

2. A compound as claimed in claim 1 wherein $R_7$ to $R_{18}$ are all hydrogen.

3. A compound as claimed in claim 1 wherein X and Y are phenyl, benzyl, phenoxy or benzyloxy, each of which may be optionally substituted with one or more of: halogen (eg. fluorine, chlorine or bromine), $C_{1-6}$alkyl [eg. methyl, ethyl, propyl (n-or iso-propyl) and butyl (n-, sec-, iso-, or tert -butyl)], $C_{1-6}$alkoxy (eg. methoxy, ethoxy, propoxy and butoxy), halo-$C_{1-6}$-alkoxy (eg. trifluoromethoxy), halo-$C_{1-6}$-alkyl (eg. trifluoromethyl), nitro, phenyl and phenoxy.

4. A compound as claimed in claim 1 or claim 2 wherein X and Y substituents are at the 2-, 3-or 4-positions of the ring, especially the 4-position.

5. A compound as claimed in any of claims 1 to 3 wherein $R^5$ and $R^6$, together with the adjacent carbon atom, represent a piperidine, morpholine, thiomorpholine, pyrrolidine or piperazine ring, any of which may be optionally substituted with one or more $C_{1-4}$ alkyl groups, phenyl or hydroxy $C_{1-4}$ alkyl.

6. A compound as claimed in any of claims 1 to 4 wherein Y is hydrogen and X is $C_{1-6}$ alkyl, $C_{3-6}$ cycloalkyl, $C_{4-9}$ cycloalkylalkyl, $C_{2-6}$ alkenyl, $C_{2-6}$ alkynyl, trimethylsilyl, triethylsilyl, t-butyldimethylsilyl, phenyldimethylsilyl, or allyldimethylsilyl.

7. A compound as claimed in any of claims 1 to 6 wherein Y is hydrogen and X is at the 3-or 4-position of the phenyl ring and is t-butyl, i-propyl or trimethylsilyl; n is 0 or 1; $R^1$, $R^2$, $R^3$ and $R^4$ are hydrogen, Q is $-CH_2-$, $-(CH_2)_2-$, $-(CH_2)_3-$or $-(CH_2)_4-$; and Z is a covalent linkage or $-CH_2-$or $-(CH_2)_2-$; and $NR^5R^6$ is 2,6-dimethylmorpholine or piperidine.

8. A process for preparing a compound as claimed in claim 1 where $R_3$ and $R_4$ are both hydrogen, which comprises treating an amide of the general formula :

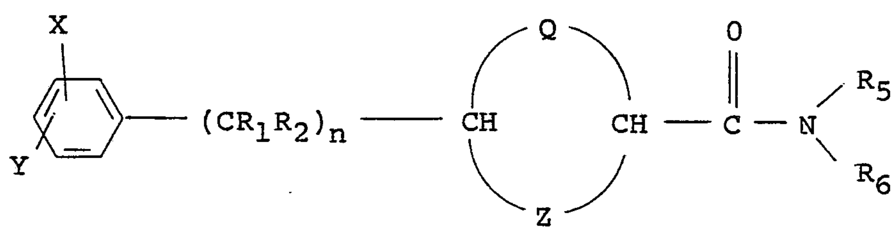

(II)

wherein $R_1$, $R_2$, $R_5$, $R_6$, Q, X, Y, Z and n are as defined in claim 1 with a reducing agent.

9. A process for preparing a compound as claimed in claim 1 which comprises treating a compound of the general formula

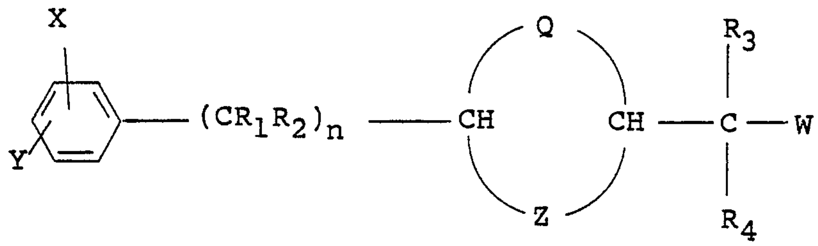

(VIII)

wherein $R_1$ to $R_4$, Q, W, X, Y, Z and n are as previously defined and W is a leaving group such as chlorine, bromine, mesylate or tosylate, with an amine of the general formula $HNR_5R_6$, where $R_5$ and $R_6$ are as defined in claim 1, optionally in the presence of a solvent.

10. A fungicidal composition comprising a compound or a salt thereof as claimed in any of claims 1 to 7 and, optionally, a carrier or diluent.

11. A method of combating fungi which comprises applying to a plant, to seed of a plant, or to the locus of the plant or seed, a compound or salt thereof as claimed in any of claims 1 to 7 or a composition as claimed in claim 10.

12. As novel intermediates the compounds of formulae (II), (III), (V), (VI), (VII), (VIII), (IX), (XI), (XII), (XVI), (XVIII) and (XXV).